(19) Europäisches Patentamt
European Patent Office
Office européen des brevets

(11) **EP 1 974 657 B1**

(12) **EUROPEAN PATENT SPECIFICATION**

(45) Date of publication and mention
of the grant of the patent:
**24.04.2013   Bulletin 2013/17**

(51) Int Cl.:
*A61B 3/135* *(2006.01)*    *A61B 3/14* *(2006.01)*

(21) Application number: **08250913.4**

(22) Date of filing: **17.03.2008**

(54) **Cornea imaging apparatus and cornea imaging method**

Hornhautbildgebungsvorrichtung und Hornhautbildgebungsverfahren

Appareil d'imagerie de la cornée et procédé d'imagerie de la cornée

(84) Designated Contracting States:
**DE IT**

(30) Priority:   **30.03.2007   JP 2007093422**

(43) Date of publication of application:
**01.10.2008   Bulletin 2008/40**

(73) Proprietor: **TOMEY CORPORATION**
**Nagoya-shi, Aichi 451-0051 (JP)**

(72) Inventors:
• **Ito, Yusaku**
**Nagoya-shi**
**Aichi 451-0051 (JP)**

• **Chihiro, Kato**
**Nagoya-shi**
**Aichi 451-0051 (JP)**

(74) Representative: **Jackson, Martin Peter**
**J A Kemp**
**14 South Square**
**Gray's Inn**
**London WC1R 5JJ (GB)**

(56) References cited:
**EP-A1- 0 628 281     WO-A1-2006/006048**
**DE-A1- 10 138 158     US-A- 5 757 461**

**Description**

BACKGROUND OF THE INVENTION

1. Field of the Invention

The present invention relates to a cornea imaging apparatus and to a cornea imaging method which involve photographing images of corneal endothelial cells by directing an illuminating light source into the eye being examined, and receiving light reflected from the cornea of the eye being examined. More particularly, the present invention is concerned with a cornea imaging apparatus and a cornea imaging method capable of photographing images of corneal endothelial cells not just in the center part of the cornea of an eye under examination, but also in peripheral parts of the cornea.

2. Description of the Related Art

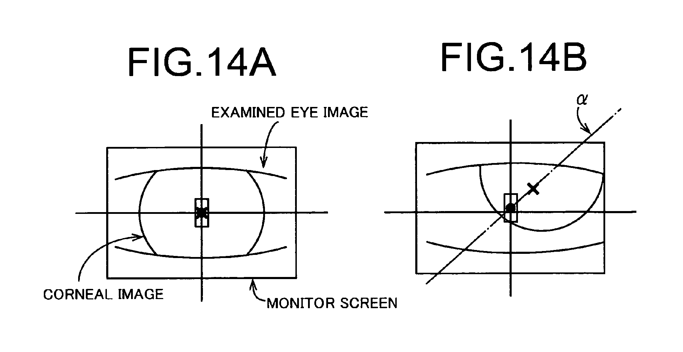

Examination of the cornea, and in particular of the condition of the corneal endothelial cells, is common practice when diagnosing ocular disease or postoperative progress of the eye.
The use of a cornea imaging apparatus capable of non-contact imaging of the corneal endothelial cells in the examined eye during such observation of the condition of the cells of the corneal endothelium is known. This cornea imaging apparatus is designed to direct a slit of illuminating light (slit beam) from an illumination optical system on the diagonal into the cornea of the examined eye, and to receive with a photographic optical system the light reflected back from the cornea in order to photograph the corneal endothelial cells.
During imaging of the corneal endothelium, it is sometimes desired to photograph not just he center part of the cornea, but also the peripheral part. This is accomplished by having the patient fix his or her gaze on the diagonal away from straight ahead, illuminating the peripheral part of the cornea with a slit of illuminating light for photographing purposes, and photographing the reflected light beam with the photographic optical system.
It will be appreciated that in such cases it may not be possible to capture a clear image of the corneal endothelium simply by having the patient fix the direction of gaze on the diagonal. This is clear for anatomical reasons, and is widely known not only by practitioners in the field of cornea imaging equipment development technology, but also by the technicians who use cornea imaging equipment. This is due to differences between the curvature of the corneal endothelium and the corneal epithelium (also known as the corneal outer envelope), whereby while in the center part of the cornea XY-alignment of the corneal endothelium can be carried out by using specular reflection by the corneal epithelium, in the peripheral part, if XY-alignment is carried out using specular reflection by the corneal epithelium in the same direction, deviation from the optimal location of the corneal endothelium will result.
Specifically, with the patient looking with a fixed gaze straight forward for the purpose of photographing the center part of the cornea, as shown in FIG. 14 (a), the optical axis of the light (shown by the $\times$ symbol) reflected by the corneal endothelium will coincide with the optical axis of the XY-alignment light (shown by the • symbol) reflected by the corneal epithelium, and thus within the photographed area indicated by the □ symbol both the corneal epithelium and the corneal endothelium will have the identical optical center axis, making it possible to carry out epithelial imaging with good accuracy through XY-alignment. However, with the patient looking with a fixed gaze diagonally for the purpose of photographing the peripheral part of the cornea, as shown in FIG. 14 (b), the optical axis of the light (shown by the x symbol) reflected by the corneal endothelium will diverge from the optical axis of the XY-alignment light (shown by the • symbol) reflected by the corneal epithelium, so even where XY-alignment is carried out, within the photographed area indicated by the □ symbol the corneal epithelium and the corneal endothelium will have different optical center axes, thus appreciably reducing the accuracy of epithelial imaging.
That is, in order to obtain a clear image of the corneal endothelium in the peripheral part of the cornea, it will be necessary to move and set the photographed area (area indicated by the □ symbol) by a prescribed distance from the center point where XY-alignment was performed (the • point) in FIG. 14(b), in the direction of deviation of the corneal endothelium (x direction).
Thus, in the past, during imaging of the peripheral part of the cornea using cornea imaging equipment, in the event that a clear image could not be photographed, the technician would set the relative location of the imaging optical system with respect to the examined eye in the XY-direction so as to be positioned offset in the appropriate direction, and take photographs repeatedly until a satisfactory image was obtained. This represented a considerable burden on both the technician and the patient, and particularly where the technician lacked experience could be extremely time-consuming, possibly subjecting the patient to considerable discomfort.
To address such problems, Patent Citation 1 (Japanese Patent No. 2812421) and Patent Citation 2 (Japanese Patent No. 3338529) proposed cornea imaging apparatuses designed such that, during XY-alignment of the imaging optical system with respect to the examined eye on the basis of an XY-alignment signal obtained from light reflected by the corneal epithelium, XY-alignment correction levels corresponding to locations of fixed gaze in diagonal directions by the examined eye are established in advance, and the XY-alignment location determined on the basis of the XY-alignment signal obtained from light reflected by the corneal epithelium is corrected by a correction level corresponding to a selected fixed gaze target.

That is, according to the technology taught in these Patent Citations 1 and 2, premised on the ability to generally identify an angle and level of correction with respect to a diagonal direction of fixed gaze by the examined eye, it is attempted to reduce the burden on the technician and the patient. It has been demonstrated that in actual practice, the direction of correction of XY-alignment, i.e. the direction of a direction of deviation line: $\alpha$ in FIG. 14 (b), can be determined with respect to the diagonal direction of fixed gaze by the examined eye on the basis of the theory of the curvature radius differential of the corneal epithelium as mentioned above; and since the correction level is be a value which will correspond to the slope angle of the axis of vision of the examined eye and the curvature radius differential between the corneal endothelium and the corneal epithelium, it is considered possible to identify from anatomical data an appropriate correction level.

However, research conducted by the inventors has shown that even where XY-alignment according to the technology taught in the prior Patent Citations 1 or 2 is employed, there are a number of cases in which the peripheral portion of the cornea still cannot be photographed with good accuracy. When the inventors examined possible reasons for this, they found that there are differences among individuals in the curvature radius of the corneal endothelium and the corneal epithelium, and that the extent of these individual differences is in some instances great enough to affect the accuracy of imaging where the correction methods taught in Patent Citation 1 or 2 are used. In particular, it should be noted that particularly patients who require imaging of the corneal endothelium should not be treated in the same way as the general populace since many of them have ocular disease or a require monitoring of post-operative progress; and it must be noted that such individuals will tend to have appreciable variability in curvature of the corneal endothelium and the corneal epithelium.

In consideration of the above, with the cornea imaging apparatuses taught in Patent Citations 1 and 2 which employ preset correction levels on the assumption, based on anatomical statistical data, that individual differences in curvature radius are not of an extent that would pose problems with imaging accuracy, correction levels during actual on-site use are sometimes inappropriate, in a relatively large number of instances ultimately requiring the technician to make fine adjustments to shooting location. In this sense, the cornea imaging apparatuses of the prior art Patent Citations 1 and 2 cannot really be considered anything more than imaging devices with a semi-automatic alignment mechanism.

To address such problems, the Applicant previously proposed an improved cornea imaging apparatus in co-pending Japanese patent application 2007-028039 (Patent Citation 3). The cornea imaging apparatus pertaining to this co-pending application photographs the corneal epithelium after performing XY-alignment with specular reflection from the corneal epithelium, then analyzes optical data of the resultant image of the corneal epithelium in order to derive from the location of XY-alignment performed on the corneal epithelium a correction level which will result in an XY-alignment location appropriate for corneal endothelium imaging.

Accordingly, in contrast to cornea imaging apparatuses such as those disclosed in the prior art Patent Citations 1 and 2 in which XY-alignment is simply corrected to a prescribed location depending on the direction of the axis of vision of the patient, with the cornea imaging apparatus taught in the co-pending application Patent Citation 3, not only can XY-alignment correction be carried out according to factors such as the corneal shape of individual patients, but XY-alignment correction can be carried out automatically, thus appreciably reducing the burden on the technician.

As a result of subsequent additional research and experimentation, the inventors arrived at a cornea imaging apparatus of novel structure and a cornea imaging method which afford an even higher level of consistency in photographing of the peripheral corneal endothelium than does the cornea imaging apparatus proposed in Patent Citation 3, and that differs fundamentally from it in terms of the basic technological concept.

EP-A-0,628,281 discloses an apparatus for observing or photographing corneal cells (such as cornea endothelium and epithelium) that provides for optical axis alignment and focusing of an imaging system (3). The apparatus is capable of assessing the position of the area to be photographed and of measuring corneal thickness from the endothelium image so produced. A whole imaging system (3) which comprises:- an indicator projecting means (23), an eye-front observation optical system (6), an illuminating system (11) for obliquely illuminating the eyeball surface (2) through a slit (16), an optical system (19) for forming an enlarged image of the subject-part by the TV camera (8), and a "corneal cell focusing" photo-detector (30). The corneal cell focusing photo-detector (30) is automatically removed in X- and Y- directions with respect to the optical axis (4), so that the light spot (40) on a monitor screen (33) is brought to a specified position on the screen. When the light spot (40) is brought to this specified position, the eye-front image is recorded and, thereafter, the imaging system (3) is started and the system (3) moves forward so as to track the light spot (40) towards the subject's eye (1). By means of the photo-detector (30), an enlarged image of the corneal cells is recorded and displayed simultaneously with the eye-front image on which the light spot (40) is positioned.

US 5,757,461 discloses a method for displaying a photographing point of a cornea which is used in an apparatus for obtaining video images of corneal cells. The apparatus includes (a) first optical assembly having a video camera provided in front of a subject eye for observing and obtaining video images of a frontal portion of the subject-eye, (b) second optical assembly for projecting slit light to an eyeball surface of the subject eye, and (c) third optical assembly for magnifying a video image of a subject portion of the eye produced based of a projecting of the slit light by the second optical assembly and for guiding the image to the video camera so that the magnified video image of the subject portion of the eye is photographed by the video camera; and the displaying method comprises the steps of (i) determining a first coordinate with respect to the video image of the frontal portion of the subject eye, which corresponds to the subject portion of the eye photographed, (ii) determining a second coordinate, which corresponds to a pupil center in the video image of the frontal portion of the subject eye; and (iii) marking and displaying the photographing point of the cornea in the video image of the frontal portion of the subject eye by calculation based upon the first and second coordinates.

SUMMARY OF THE INVENTION

The Applicant previously proposed in a co-pending application (Patent Citation 3) a cornea imaging apparatus equipped with a novel automatic XY-alignment correction mechanism for use during photographing or imaging of the peripheral corneal endothelium, representing an advance over the conventional cornea imaging apparatuses taught in Patent Citations 1 and 2 which were equipped with a semi-automatic XY-alignment correction mechanism. In view of the foregoing it is now an object of the present invention to provide a further improved cornea imaging apparatus which affords a higher level of consistency and better accuracy of XY-alignment correction during photographing of the peripheral corneal endothelium than does the cornea imaging apparatus proposed in the co-pending application (Patent Citation 3), whereby it is possible to further reduce the burden on the technician and the patient during actual on-site use and to make imaging faster.

The modes of the present invention for the purpose of addressing this problem will be described below. The constituent elements employed in the modes herein may be employed in any number of possible combinations.

Specifically, the present invention in the aspect thereof relating to a cornea imaging apparatus is provided in accordance with claim 1 appended hereto.

That is, the XY-alignment procedure during corneal imaging is derived from the original prior art technology whereby the technician carries out the procedure using reflection from the epithelium of the examined eye. The inventions pertaining to Patent Citations 1 through 3 teach technological concepts that do not depart from the framework of this conventional basic technological concept of performing XY-alignment on the basis of reflected light from the corneal epithelium. This conventional technological concept is based on the fact that the corneal epithelium is directly viewable from the outside, and that due to the significantly larger quantity of light gathering reflected light from the corneal epithelium is easier than gathering reflected light from the corneal endothelium; and additionally on the fact that, at least at the cornea center (on the visual axis), the orthogonal axes (frontal reflection axes) of the corneal epithelium and the corneal endothelium generally coincide, so that in corneal endothelium imaging, the majority of which involves photographing the cornea center, the technician will be able to ascertain frontal reflection from the corneal epithelium, and perform XY-alignment on the basis of this frontal reflection from the corneal epithelium.

In the present invention however, this conventional technological concept of XY-alignment is disregarded entirely, and instead based on the entirely novel technological concept of performing XY-alignment with the corneal endothelium from the outset. Accordingly, the XY-alignment technology taught herein differs fundamentally from the conventional one.

According to the present invention wherein XY-alignment is carried out utilizing optical information of the corneal endothelium:

   (1) rather than "simply establishing a correction level for XY-alignment at a prescribed location (i.e. a specified (Note: definition from Kojien) location) corresponding to the location of a fixed gaze target (i.e. the direction of the visual axis of the patient)" as with the cornea imaging apparatuses taught in the aforementioned prior art Patent Citations 1 and 2, "each time that the location of the patient or of the fixed target changes, an optimal location of XY-alignment is individually measured, computed, and determined"; and moreover,

   (2) rather than determining XY-alignment of the corneal endothelium in a presumptive manner from optical information of the corneal epithelium as with the cornea imaging apparatus proposed in co-pending Patent Citation 3, XY-alignment of the corneal endothelium is determined directly from optical information of the corneal endothelium. Thus, according to the cornea imaging apparatus pertaining to the present invention:

      (i) in contrast to the cornea imaging apparatuses taught in the aforementioned prior art Patent Citations 1 and 2, lower measurement accuracy caused by individual differences among individual patients can be avoided to the greatest extent practical, making it possible to consistently obtain corneal endothelium images with good accuracy, and thus in contrast to the semi-automatic type of cornea imaging apparatus taught in the aforementioned prior art Patent Citations 1 and 2, there is afforded a truly automatic imaging device that reduces the burden on the technician and the patient; moreover,

(ii) as compared with the cornea imaging apparatus taught in co-pending Patent Citation 3, by using optical information of the corneal endothelium, XY-alignment can be carried out with a higher level of accuracy during corneal endothelium imaging; and the desired corneal endothelium images can be obtained quickly and easily regardless of differences in technical skills or experience of the technician, thereby appreciably reducing the burden on the technician and the patient.

In the present invention, as the corneal endothelium optical information acquired by the XY-alignment optical information acquisition control means it would be possible to employ either the optical information of the XY-alignment corneal endothelium image taken by the cornea imaging optical system; or sensor values of the quantity of reflected light from the corneal endothelium measured using the Z-direction location observation photoreceptor element.

In the present invention, configurations such as the following may be employed, for example. Specifically, in the present invention it is possible to employ a configuration wherein the aforementioned "XY-alignment optical information acquisition control means" acquires the corneal endothelium optical information on the optical axis of the frontal reflection from the corneal epithelium of the examined eye of the XY-alignment pointer light directed thereon by the XY-alignment pointer light emitting means; and on the basis of the acquired corneal endothelium optical information, the XY-alignment reference location determining means determines the XY-alignment reference location on a prescribed XY-direction line which corresponds to a diagonal direction of the visual axis (Mode i).

By way of a more specific configuration in Mode i, it is possible to employ a configuration wherein, for example, an image of the corneal endothelium for XY-alignment purposes is photographed by the cornea imaging optical system on the optical axis of frontal reflection from the corneal epithelium of the examined eye of the XY-alignment pointer light; luminance level is obtained by way of optical information for the acquired XY-alignment corneal endothelium image. A boundary location of a pre-established luminance level value or contrast value in the acquired image is derived through an arithmetic operation; and analyzing where in the XY-alignment corneal endothelium image the boundary location is, curvature of the corneal endothelium is estimated, in order to determine a reference location for XY-alignment. In other words, it is possible to employ a configuration involving estimating how far away the location of the reference location for XY-alignment is in the XY-direction with respect to the optical axis of frontal reflection from the corneal epithelium of the examined eye of the XY-alignment pointer light. The optical axis of frontal reflection from the corneal epithelium of the examined eye of the XY-alignment pointer light will be an orthogonal axis to the corneal epithelium.

In the aforementioned Mode i, when determining the direction of the XY-alignment reference location with respect to the optical axis of frontal reflection from the corneal epithelium of the examined eye of the XY-alignment pointer light, it would be possible, for example, to make the determination in correspondence with the location of the peripheral fixed gaze target employed, in other words, with the diagonal direction of the visual axis of the examined eye. Alternatively, it would be possible to make the determination, for example, on the basis of a distribution of luminance level values or contrast values for the aforementioned XY-alignment corneal endothelium image obtained by imaging on the optical axis of frontal reflection from the corneal epithelium of the examined eye of the XY-alignment pointer light (as a specific example, relative location of areas of high and low luminance level to either side of a prescribed luminance value boundary line).

Furthermore, in the aforementioned Mode i, it would be possible to employ XY-alignment reference location verification means that, after the XY-alignment reference location has been determined, moves and positions the illumination optical system and the cornea imaging optical system to the determined XY-alignment reference location, and with these positioned at the XY-alignment reference location, reacquires the corneal endothelium optical information using the XY-alignment optical information acquisition control means, and verifies the XY-alignment reference location on the basis of the corneal endothelium optical information acquired thereby.

By employing such verification means, even where XY-alignment reference location is being determined on the basis of minimal optical information (e.g. a single image of the corneal endothelium for XY-alignment), it will be possible to ensure reliability and accuracy of the determined location. Put the opposite way, it will be possible to efficiently determine XY-alignment reference location from minimal optical information while ensuring high reliability and accuracy. It would be possible to carry out such verification, for example, using as an indicator the value of the luminance level or contrast distribution in the XY-alignment corneal endothelium image photographed while positioned at the XY-alignment reference location.

If the results of verification are not valid, this can be dealt with be again acquiring corneal endothelium optical information such as an image of the corneal endothelium for XY-alignment, and repeatedly determining an XY-alignment reference location. In this case, the image of the corneal endothelium for XY-alignment or other such corneal endothelium optical information may be acquired, for example, at a location situated away in the XY-direction from the optical axis of frontal reflection from the corneal epithelium of the examined eye of the XY-alignment pointer light.

In the present invention, besides a configuration in which XY-alignment is performed utilizing corneal endothelium optical information acquired on the optical axis of frontal reflection from the corneal epithelium of the examined eye of the XY-alignment pointer light, it would be possible to employ a configuration such as the following, for example. Specifically, in the present invention, there may be employed a configuration wherein the XY-alignment optical information acquisition control means, using the XY-direction actuating means, moves the illumination optical system and the cornea imaging optical system as a unit in a prescribed XY-direction which corresponds to the diagonal direction of the visual axis, and acquires the corneal endothelium optical information at multiple sites on a line in the prescribed XY-direction (Mode ii).

Through acquisition of multiple observations of corneal endothelium optical information in this way and use of these optical information observations, it is possible through verification or rechecking using discrete corneal endothelium optical information observations to determine the desired XY-alignment reference location with a higher level of accuracy as compared to the case where an XY-alignment reference location is derived on the basis of only a single observation of XY-alignment corneal endothelium optical information; and to thereby more consistently obtain highly accurate corneal endothelium images. Moreover, by comparing multiple observations of corneal endothelium optical information photographed while moving in the XY-direction and analyzing the extent of change associated with movement in the XY-direction in order to derive the curvature of the corneal endothelium, it is possible to more efficiently determine an XY-alignment reference location.

Furthermore, in Mode ii which involves acquisition of corneal endothelium optical information at multiple sites on a line in a prescribed XY-direction, it is possible to concomitantly employ the configuration of (I) or (II) below, for example.

(I) A configuration whereby in the XY-alignment reference location determining means,
acquisition of the corneal endothelium optical information by the XY-alignment optical information acquisition control means on the line in the prescribed XY-direction is carried out until and terminated at a pre-established end location.
(II) A configuration whereby in the XY-alignment reference location determining means,
each time that the corneal endothelium optical information is acquired on the line in the prescribed XY-direction by the XY-alignment optical information acquisition control means, sequential evaluation is performed on the basis of the acquired corneal endothelium optical information; and when it is verified that an evaluation result meets a predetermined condition established in advance the XY-alignment reference location is determined and acquisition of the corneal endothelium optical information terminates.

In the present invention, it is possible to employ a line sensor as the Z-direction location observation photoreceptor element. Specifically, while it is acceptable for the Z-direction location observation photoreceptor element to simply sense the presence or absence of focus using a photodiode, by employing a line sensor it will be possible to additionally ascertain a location forward or backward in the Z-axis direction that will afford focus, making it possible to consistently focus in the Z-axis direction with high accuracy. Consequently, during acquisition of corneal endothelium optical information for XY-alignment purposes, more highly accurate positioning of the illumination optical system and the cornea imaging optical system at a prescribed location in the Z-direction (a location past the focal location on the corneal epithelium and closer to the corneal endothelium) will be possible with XY-alignment Z-direction location control means which utilize the output of the line sensor.

The Z-direction location observation photoreceptor element in the present invention many be any element capable of observing the condition of focus in the Z-direction. It is not necessary limited to one that senses focal location of the corneal endothelium, and of course is not limited to the line sensor mentioned above. As a specific example, it is acceptable for the element to sense only the epithelial focal location, in which case the endothelial focal location may be estimated on the basis of the epithelial focal location (for example, see the known art disclosed inter alia in JP-A-6-63018 and JP-A-8-71043). Alternatively, it is possible to carry out location observation in the Z-direction of the condition of focus on the corneal epithelium or the corneal enbothelium by employing the imaging results of continuous photographing means in the Z-direction.

The following configuration may be advantageously employed in the aforementioned Mode ii which involves acquisition of corneal endothelium optical information at multiple sites on a line in a prescribed XY-direction. Specifically, it is possible to employ a configuration wherein a line sensor is employed as the Z-direction location observation photoreceptor element, the XY-alignment optical information acquisition control means acquires corneal endothelium optical information for XY-alignment use from the line sensor, and the XY-alignment optical information acquisition control means designates the location of maximum quantity of reflected light in the line sensor as the XY-alignment reference location (Mode iii).

In Mode iii, quantity of light distribution information in the Z-direction, inclusive of information to the front and back of the corneal endothelium, can be obtained by way of corneal endothelium optical information for XY-alignment purposes. Specifically, it will be possible to easily acquire reflected quantity of light distribution information through an area of several hundred $\mu$m in the Z-direction. Consequently, during acquisition of corneal endothelium optical information for XY-alignment purposes, location setting accuracy in the Z-direction with respect to the examined eye may be looser, making it easier to use the device and further reducing the labor and discomfort on the part of the technician and patient. Moreover, it will be possible to consistently acquire XY-alignment corneal endothelium optical information even where the location of sensed corneal endothelium-reflected light differs in the Z-direction due to variability in cornea thickness among individual patients.

In particular, as compared to the case where a corneal endothelium image is used as XY-alignment corneal endothelium optical information, where quantity of light distribution information sensed by a line sensor is employed as the XY-alignment corneal endothelium optical information the accuracy of alignment of the device in the Z-direction with respect to the examined eye during acquisition of XY-alignment corneal endothelium optical information may typically be lower.

During acquisition of XY-alignment corneal endothelium optical information using a line sensor as the Z-direction location observation photoreceptor element, and subsequent derivation of the location of the optical axis of frontal reflection from the corneal endothelium in the XY-direction on the basis of the result, when determining the XY-alignment reference location on the basis of corneal endothelium optical information acquired at multiple locations as described above, it will be possible also to efficiently carry out correction of errors caused by Z-direction displacement occurring due to change in cornea curvature at the multiple locations.

There may be advantageously employed a configuration for example, provided with optical information correction level computing means that during error correction uses the line sensor in the XY-alignment optical information acquisition control means to acquire corneal epithelium optical information in conjunction with acquisition of corneal endothelium optical information for XY-alignment purposes, and on the basis of the corneal epithelium optical information calculates a correction level for an error in the corneal endothelium optical information due to change in relative location in the Z-direction of the illumination optical system and the cornea imaging optical system with respect to the examined eye; and with optical information correction executing means that corrects the XY-alignment corneal endothelium optical information acquired by the line sensor, using the correction level derived by the optical information correction level computing means.

Alternatively, in addition to error correction of acquired corneal endothelium optical information or in place of such error correction, there may be advantageously employed a configuration such as the following, for the purpose of correcting error caused by Z-direction displacement. Specifically, there may be advantageously employed a configuration provided with Z-direction change computing means that during acquisition of corneal endothelium optical information for XY-alignment purposes by the line sensor in the XY-alignment optical information acquisition control means acquires corneal epithelium optical information with the line sensor, and on the basis of the corneal epithelium optical information calculates change of relative location in the Z-direction of the illumination optical system and the cornea imaging optical system with respect to the examined eye; and with Z-direction location correction executing means that performs correction of relative location of the illumination optical system and the cornea imaging optical system with respect to the examined eye in the Z-direction, using as the correction level the change of relative location in the Z-direction calculated by the Z-direction change computing means. Specifically, with such Z-direction location correction executing means, for example during movement of an optical system in the XY-direction with respect to the examined eye while sensing reflected light from the corneal endothelium, by performing Z-direction location correction of the optical system in question with respect to the examined eye in such a way that a location of reflected light from the corneal epithelium (e.g. the location of the greatest value of reflected light from the corneal epithelium) will be positioned within a given reference range on the line sensor, it is possible to avoid Z-direction deviation from going above a prescribed value and excessively large measurement error.

In particular, according to the former mechanism for error correction of acquired corneal endothelium optical information, even if Z-direction alignment error is present, accurate corneal endothelium optical information can still be acquired quickly without the need to reposition the illumination optical system or cornea imaging optical system in the Z-direction with respect to the examined eye.

According to the latter mechanism for error correction of acquired corneal endothelium optical information, at each location for acquiring corneal endothelium optical information, it will be possible to position the illumination optical system or cornea imaging optical system in the Z-direction with respect to the examined eye with a high degree of accuracy prior to acquiring corneal endothelium optical information, making it possible to directly acquire corneal endothelium optical information with consistently high accuracy using the XY-alignment optical information acquisition control means.

The former mechanism for error correction of corneal endothelium optical information is particularly advantageous for relatively small displacement in the Z-direction, whereas the latter mechanism for error correction of corneal endothelium optical information can deal with relatively large displacement in the Z-direction as well. Consequently, appropriate use of either of these correction mechanisms, or concomitant use of both is acceptable.

Where the former mechanism for error correction of corneal endothelium optical information is employed, it will be necessary to know the correlation relationship between change in the quantity of light reflected from the corneal endothelium and change in the quantity of light reflected from the corneal epithelium in the line sensor during displacement in the Z-direction; this correlation relationship can be derived through experimentation or trial runs, or calculated statistically. By deriving the correlation relationship between the two in advance, the correction level computing process can be carried out faster.

Since the quantity of light reflected from the corneal epithelium is very large compared to the quantity of light reflected from the corneal endothelium, it may occur that the quantity of light reflected from the corneal epithelium will reach saturation over a prescribed location range in the Z-direction, so that an unchanging value for sensed quantity of reflected light is observed. Through prior consideration of line sensor characteristics it will be possible to deal with such instances by not performing correction in the area which gives this saturation sensor value.

Furthermore, during measurement of the quantity of light reflected from the corneal endothelium using a line sensor as described above, it would be possible to irradiate the examined eye with an infrared beam and measure the quantity of reflected light. By employing such a configuration it will be possible to minimize increased burden on the patient while advantageously acquiring corneal endothelium optical information for XY-alignment purposes. Moreover, in the present mode, since acquisition of corneal endothelium optical information for XY-alignment is carried out utilizing a line sensor as the Z-direction location observation photoreceptor element, it will be possible to carry out highly accurate XY-alignment while avoiding an excessively complex structure.

In the present invention, it is also possible to employ a configuration such as the following, for example. Specifically, in the present invention, there can be employed a configuration wherein the corneal endothelium photographing image control means continuously photographs the corneal endothelium images at multiple sites along a line in the Z-direction while moving the illumination optical system and the cornea imaging optical system in the Z-direction relative to the examined eye (Mode iv).

By acquiring multiple corneal endothelium images on a line in the Z-direction as in Mode iv, it will be possible to select the best image from among the multiple corneal endothelium images, making it possible as a result to more consistently obtain the desired corneal endothelium images of high quality. Configurations for continuous imaging include photographing while the optical system travels continuously with respect to the examined eye; and photographing while the optical system is moved in stepwise fashion in prescribed distance increments with respect to the examined eye.

In Mode iv, it will be possible to concomitantly employ the following configuration. Specifically, in Mode iv there may be advantageously employed a configuration provided with XY-alignment correction control means that, during continuous photographing of corneal endothelium images at multiple sites along a line in the Z-direction, utilizes intervals between photographs in order to carry out photoreception of frontal reflection from the corneal epithelium with the XY-alignment pointer light emitting means and the XY-alignment photoreceptor element and calculate an orthogonal axis to the corneal epithelium, as well as to control the XY-direction actuating means while making allowance for the orthogonal axis to the corneal epithelium; and that performs position correction such that the illumination optical system and the cornea imaging optical system are positioned at the XY-alignment reference location determined by the XY-alignment reference location determining means.

In Mode iv concomitantly employing the above configuration, it will be possible to use the readily observable optical axis of frontal reflection from the corneal epithelium as a reference axis to align the illumination optical system and the cornea imaging optical system with the XY-alignment reference location. Once the XY-alignment reference location has been determined, even where multiple images of the corneal endothelium are being photographed, it will subsequently be easy to perform repeated adjustment to align the illumination optical system and the cornea imaging optical system with the XY-alignment reference location, as long as the optical axis of frontal reflection from the corneal epithelium can be observed.

The present invention in another aspect thereof relating to a cornea imaging method as defined in appended claim 12.

According to this method of the present invention, data acquired directly from the corneal endothelium (corneal endothelium optical information for XY-alignment purposes) can be utilized to calculate an orthogonal axis to the corneal endothelium, whereby the cornea imaging optical system can be aligned with high accuracy on the orthogonal axis to the corneal endothelium in the examined eye, thereby making it possible to consistently and photograph the desired accurate images of the peripheral portion of the corneal endothelium.

By employing a configuration comprising (e) through (g) in combination with a configuration comprising (a) through (d), it will be possible to more reliably obtain the desired images of the peripheral portion of the corneal endothelium.

BRIEF DESCRIPTION OF THE DRAWINGS

The foregoing and/or other objects features and advantages of the invention will become more apparent from the following description of a preferred embodiment with reference to the accompanying drawings in which like reference numerals designate like elements and wherein:

FIG. 1 is a view for explaining an apparatus optical system of a cornea imaging apparatus according to a first embodiment of the present invention;

FIG. 2 is a view for explaining a fixation target (fixed gaze lamp) arrangement in the apparatus optical system according to the first embodiment of the invention;

FIG. 3 is a view for explaining a cornea imaging apparatus of the first embodiment;

FIG. 4 is a view for explaining control circuits or the like connectable to the optical system shown in FIG. 1;

FIG. 5 is a flow chart demonstrating basic imaging processes executed by the cornea imaging apparatus;

FIG. 6 is a view for explaining an image of an examined eye shown on a monitor screen;

FIG. 7 is a flow chart demonstrating processes including XY-alignment according to the first embodiment of the present invention;

FIG. 8 is a view for explaining operation for determining reference location according to the flow chart of FIG. 7;

FIG. 9 is a graph demonstrating a light intensity distribution of reflected beams detected at each location (P) of FIG. 8;

FIG. 10 is a graph demonstrating a change in detected values of reflected beams based on an amount of deviation in the Z-direction upon detecting reflected beams of cornea;

FIG. 11 shows specific images of a corneal endothelium obtainable at each point (P) in FIG. 8;

FIG. 12 is a graph demonstrating a comparison in terms of light quantity of detected reflected beams of cornea at each location (P) of FIG. 8;

FIG. 13 is a flow chart demonstrating processes including XY-alignment according to a third embodiment of the present invention; and

FIG. 14 is a view for explaining problems to be solved in a cornea endothelium imaging apparatus.

## DETAILED DESCRIPTION OF PREFERRED EMBODIMENTS

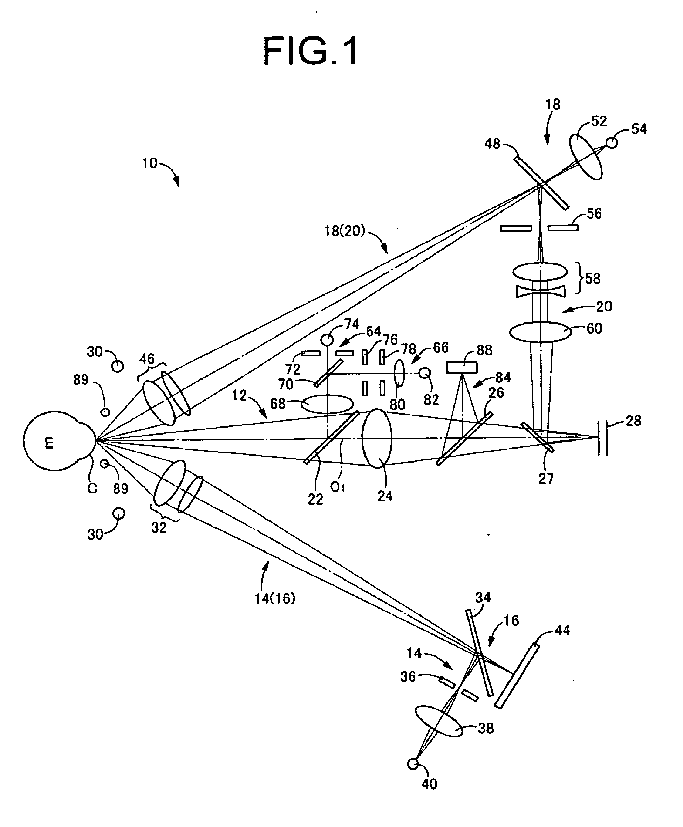

First, FIG. 1 shows a cornea imaging apparatus pertaining to a first embodiment of the present invention. In order to facilitate understanding of its configuration, in the drawing the cornea imaging apparatus is shown as an apparatus optical system 10 with the console etc. removed.

In the apparatus optical system 10, to one side of an observation optical system 12 for observing the anterior of the examined eye E, there are disposed an imaging illumination optical system 14 as the illumination optical system and a location sensing optical system 16; and to the other side are disposed location sensing illumination optical system 18 and an imaging optical system 20 as the cornea imaging optical system.

The observation optical system 12 is composed, in order from the location closest the examined eye E, of a half mirror 22, an objective lens 24, a half mirror 26, a cold mirror 27, and a CCD 28 as the photoelectric element, disposed on the optical axis Ol. A plurality of observation light sources 30, 30 (two in the present embodiment) are positioned in front of the examined eye E. The observation light sources 30, 30 may employ infrared LEDs that emit a beam of infrared light, for example. The cold mirror 27 allows infrared light to pass while reflecting visible light; beams of light emitted by the observation light sources 30, 30 and reflected from the anterior of the examined eye E will pass through the objective lens 24 and the cold mirror 27, and become focused on the CCD 28.

The imaging illumination optical system 14 is composed, in order from the location closest the examined eye E, of a projector lens 32, a cold mirror 34, a slit 36, a collecting lens 38, and a imaging light source 40. The imaging light source 40 serving as the illumination light source may employ an LED that emits a beam of visible light, for example. The cold mirror 34 allows infrared light to pass while reflecting visible light. The beam of light emitted by the imaging light source 40 passes through the collecting lens 38 and the slit 36 where it becomes a slit beam, and after being reflected by the cold mirror 34 passes through the projector lens 32 and is directed onto the cornea C from a diagonal direction.

The location sensing optical system 16 is situated with part of its optical axis aligned with the optical axis of the imaging illumination optical system 14 and is composed, in order from the location closest the examined eye E, of a projector lens 32, a cold mirror 34, and a line sensor 44 as the Z-direction location observation photoreceptor element. A beam of light emitted by a location sensing light source 54, described later, and reflected from the cornea C will pass through the projector lens 32 and the cold mirror 34, and become focused on the line sensor 44.

The location sensing illumination optical system 18, meanwhile, is composed, in order from the location closest the examined eye E, of an objective lens 46, a cold mirror 48, a collecting lens 52, and the location sensing light source 54. The location sensing light source 54 preferably employs an infrared light source such as an infrared LED, for example. The beam of infrared light emitted by the location sensing light source 54 is directed on the diagonal onto the cornea C. The location sensing light source 54 may also be composed of a combination of a visible light source, such as a halogen lamp or visible light LED, with an infrared filter. However, it is not always necessary for the location sensing light source 54 to be an infrared light source, and a visible light source such as a halogen lamp or visible light LED could be used instead. Where a visible light source is employed, it is preferable for its illuminance to be lower than the illuminance of the imaging light source 40. This can reduce the burden on the patient when a beam of light from the location sensing light source 54 is directed into the eye during alignment and so on.

The imaging optical system 20 serving as the cornea imaging optical system is situated with part of its optical axis aligned with the optical axis of the location sensing illumination optical system 18 and is composed, in order from the location closest the examined eye E, of an objective lens 46, a cold mirror 48, a slit 56, a variable power lens 58, a focusing lens 60, a cold mirror 27, and a CCD 28. A beam of light emitted by the imaging light source 40 and reflected from the cornea C will be reflected by the cold mirror 48 via the objective lens 46, then transformed into a parallel beam by the slit 56, reflected from the cold mirror 27 via the variable power lens 58 and the focusing lens 60, and focused on the CCD 28.

The half mirror 22 disposed in the observation optical system 12 also constitutes part of a center fixed gaze target optical system 64 and an alignment optical system 66.

The center fixed gaze target optical system 64 is composed, in order from the location closest the examined eye E, of a half mirror 22, a projector lens 68, a half mirror 70, a pinhole plate 72, and a center fixed gaze target light source 74. The center fixed gaze target light source 74 light source may employ a light source that emits a beam of visible light, such as a LED for example. A beam of light emitted by the center fixed gaze target light source 74 will pass through the pinhole plate 72 and the half mirror 70, then be transformed to a parallel beam by the projector lens 68, and reflected into the examined eye E by the half mirror 22.

The alignment optical system 66 is composed, in order from the location closest the examined eye E, of a half mirror 22, a projector lens 68, a half mirror 70, an aperture diaphragm 76, a pinhole plate 78, a collecting lens 80, and an alignment light source 82. The alignment light source 82 emits infrared light serving as pointer light for XY-alignment. The infrared light is collected by the collecting lens 80, passes through the pinhole plate 78, and is guided into the aperture diaphragm 76. Light passing through the aperture diaphragm 76 is reflected by the half mirror 70, transformed into a parallel beam by the projector lens 68, then reflected into the examined eye E by the half mirror 22. The XY-alignment pointer light emitting means is constituted by this beam emitting mechanism for the examined eye E.

The half mirror 26 disposed on the observation optical system 12 constitutes part of an alignment sensing optical system 84 for XY-alignment.

The alignment sensing optical system 84 is composed, in order from the location closest the examined eye E, of a half mirror 26 and an alignment sensor (profile sensor) 88 able to sense location. The system is designed so that light emitted by an alignment light source 82 is incident on the cornea C of the examined eye E along the optical axis O1 of the observation optical system 12 coincident with the axis of frontal vision of the examined eye E. The specular reflected beam from the cornea C travels as-is along the same optical axis O1 as the illuminating light from the alignment light source 82 and is reflected by the half mirror 26, and guided onto the alignment sensor 88 which serves as the XY-alignment photoreceptor element.

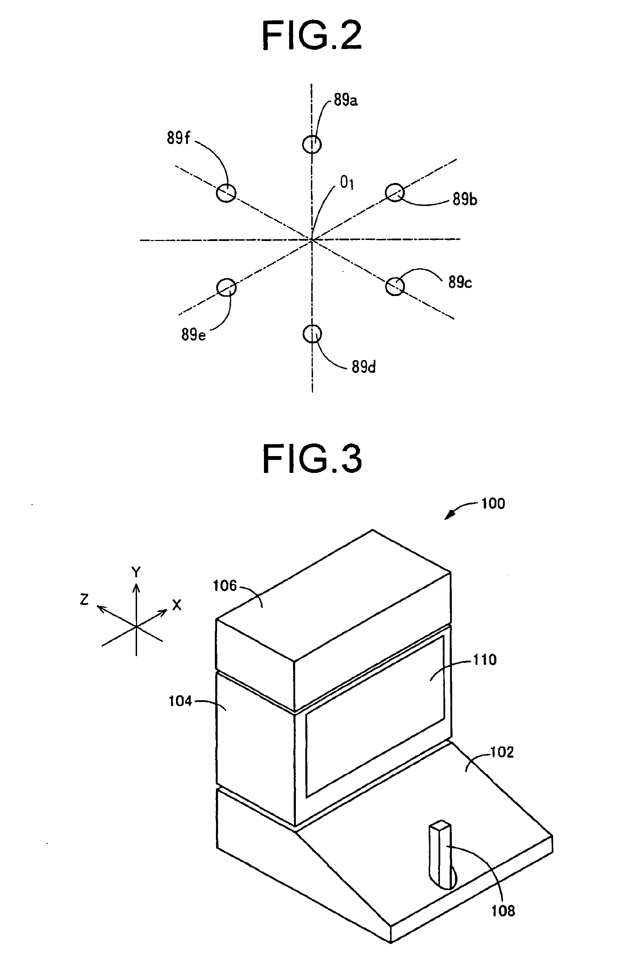

Additionally, a plurality of fixed gaze lamps 89 serving as peripheral fixed gaze targets are positioned in appropriate directions about the optical axis O1 of the observation optical system 12 which is coincident with the axis of frontal vision of the examined eye E, at locations such that the optical paths of the optical systems discussed above are not affected. Specifically, as depicted in FIG. 2 for example, the fixed gaze lamp 89 arrangement may be composed of a total of six fixed gaze lamps 89a, 89b, 89c, 89d, 89e and 89f situated on a hypothetical clock face at positions corresponding to 12 o-clock at top and 6 o-clock at bottom, and in the left and right halves at positions corresponding to 2 o-clock, 4 o-clock, 8 o-clock, and 10 o-clock, at equidistant intervals along a single circle having the optical axis O1 as its center axis. The fixed gaze lamps 89 may employ visible LEDs or the like, so as to be visible to the patient.

The plurality of fixed gaze lamps 89a through 89f are designed to be alternatively selectively illuminated during photographing of the peripheral part of the cornea C. By having the patient fix his or her gaze onto any one illuminated fixed gaze lamp 89, a specific peripheral part of the cornea C may be positioned in a zone illuminated by a slit beam and photographed by the cornea imaging optical system, in other words, positioned on the optical axis O1 of the observation optical system 12.

The apparatus optical system 10 having a structure such as that described above is housed within a cornea imaging apparatus 100 such as shown in FIG. 3 for example. The cornea imaging apparatus 100 is composed of a main unit 104 disposed on top of a base 102. A case 106 is disposed moveably front to back, left to right, and up and down on top of the main unit 104. The base 102 houses the power supply and is also provided with a control stick 108, and the case 106 can be actuated through operation of the control stick 108. Various control circuits, to be discussed later, are also housed within the main unit 104, and a display screen 110 such as an LCD monitor is provided as well.

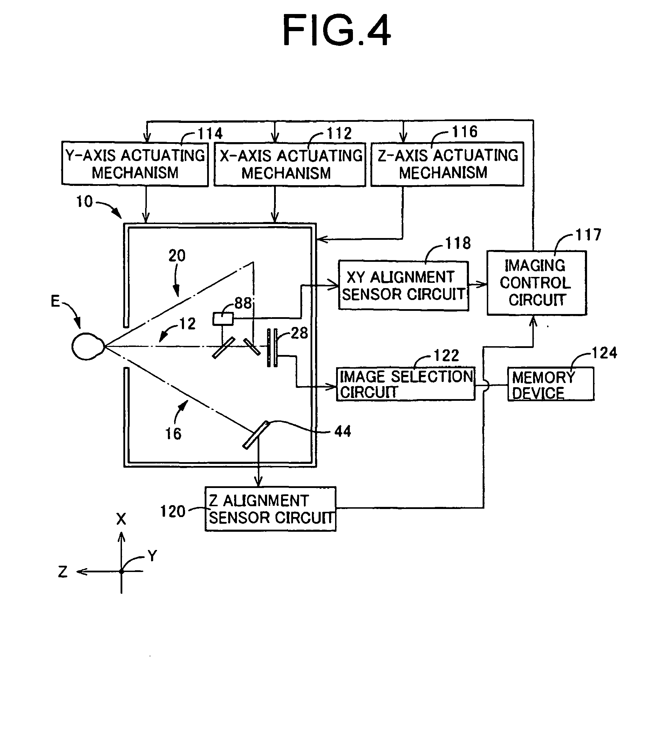

As shown in FIG. 4, the cornea imaging apparatus 100 is equipped with actuating means for actuating the case 106 in order to move the apparatus optical system 10 in a direction closer to or away from the examined eye E. The actuating means may be composed, for example, of rack and pinion mechanisms or the like. In the present embodiment, there are provided an X-axis actuating mechanism 112 for actuating the apparatus optical system 10 in the X-direction which is the vertical direction in FIG. 4. A Y-axis actuating mechanism 114 for actuating it in the horizontal Y-direction which is perpendicular to the plane of the paper. A Z-axis actuating mechanism 116 for actuating it in the horizontal Z-direction which is sideways in FIG. 4 (i.e. the direction along the optical axis O1 of the observation optical system 12).

The cornea imaging apparatus 100 is equipped with an imaging control circuit 117 as imaging control means for operational control of photography of corneal images by the apparatus optical system 10. The X-axis actuating mechanism 112, the Y-axis actuating mechanism 114, and the Z-axis actuating mechanism 116 are connected respectively to the imaging control circuit 117 so as to be actuated on the basis of actuating signals from the imaging control circuit 117. The alignment sensor 88 is connected to an XY-alignment sensor circuit 118, and this XY-alignment sensor circuit 118 is in turn connected to the imaging control circuit 117. The line sensor 44 is connected to a Z alignment sensor circuit 120, and this Z alignment sensor circuit 120 is in turn connected to the imaging control circuit 117. Sensor information from the alignment sensor 88 and from the line sensor 44 can thereby be input to the imaging control circuit 117. While not shown in the drawing, the imaging control circuit 117 is also connected to the illuminating light sources 30, 40, 54, 74, and 82, and can control their light emission.

The cornea imaging apparatus 100 is also furnished with an image selection circuit 122 for selecting or discarding images captured by the CCD 28 when these images are input to the device; and a storage device 124 serving as storage means for saving images selected by the image selection circuit 122.

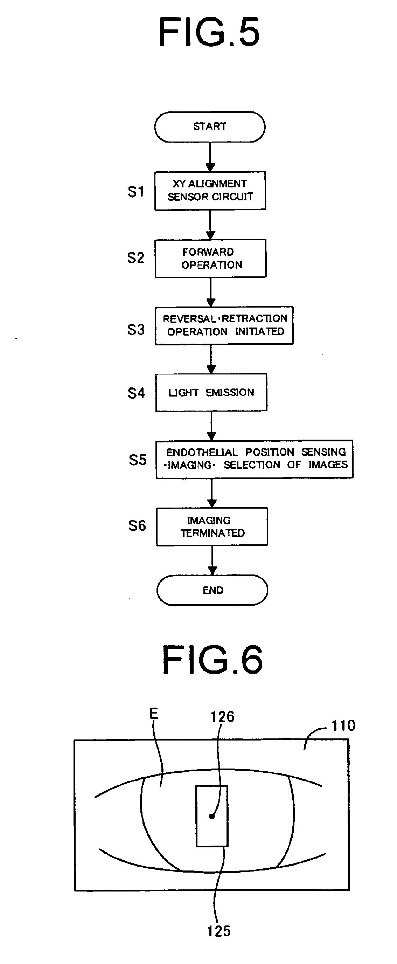

Next, an overview of the procedure executed by the imaging control circuit 117 to photograph the corneal endothelium in the cornea imaging apparatus 100 of the structure described above is shown in FIG. 5, and will be described in sequence hereinbelow.

The description turns first to the case of photographing the endothelium at the center of the cornea of the examined eye E.

First, in S1, the alignment of the apparatus optical system 10 in the X-direction and the Y-direction (XY-alignment) is performed. During this XY-alignment, fixed gaze target light from the center fixed gaze target light source 74 is directed into the examined eye E. Then, by having the patient fix his or her gaze on the fixed gaze target light, the direction of the optical axis of the examined eye E can assume the frontal vision state aligned with the direction of the optical axis O1 of the observation optical system 12. In this state, beams of light emitted by the observation light sources 30, 30 and reflected from the anterior part of the examined eye E will be directed onto the CCD 28. As shown in FIG. 6, an image of the anterior part of the examined eye E will thereby be displayed on the display screen 110. For XY-alignment in this case, since the eye is in the frontal vision state, normal lines perpendicular to tangent lines of the corneal endothelium and of the epithelium can considered to have identical direction (i.e. identical direction of a center axis which is an extension of the curvature radius (line of vision)). It will therefore suffice to perform XY-alignment on the basis of reflected light from the corneal epithelium (i.e. it may be considered that XY-alignment of the corneal endothelium is possible thereby).

Additionally, an alignment pattern 125 of rectangular frame shape generated by a superimposed signal for example is shown superimposed on the examined eye E on the display screen 110. At the same time, the beam of light beamed towards the examined eye E by the alignment light source 82 will be reflected by the anterior part of the examined eye E and directed onto the CCD 28, so that the alignment light 126 will be displayed in point form on the display screen 110. Then, by operating the control stick 108 the operator will actuate the apparatus optical system 10 and adjust the location of the apparatus optical system 10 so that the alignment light 126 is positioned within the frame of the alignment pattern 125.

Part of the beam emitted from the alignment light source 82 and reflected from the anterior part (corneal epithelium) of the examined eye E will be reflected by the half mirror 26 and directed onto the alignment sensor 88. The burden on the patient is reduced by having the alignment light source 82 emit a beam of infrared light not noticeable to the patient. Here, once the alignment light 126 enters the frame of the alignment pattern 125, the alignment sensor 88 will be able to sense the X-direction location and the Y-direction location of the alignment light 126. This X-direction location and Y-direction location will be input to the XY-alignment sensor circuit 118. The XY-alignment sensor circuit 118, on the basis of the X-direction location information, will actuate the X-axis actuating mechanism 112 to bring the optical axis OA of the observation optical system 12 close to the optical axis of the examined eye E, and on the basis of the Y-direction location information will actuate the Y-axis actuating mechanism 114 to bring the optical axis OA of the observation optical system 12 close to the optical axis of the examined eye E. From the above description it will be appreciated that, in the present embodiment, the XY-direction actuating means includes the X-axis actuating mechanism 112 and the Y-axis actuating mechanism 114.

As will be discussed later, this XY-alignment is carried out at appropriate timing during imaging as well. In the present embodiment in particular, the alignment light source 82 and the observation light sources 30, 30 alternately go on and off for brief intervals, and sensing by the alignment sensor 88 is carried out at timing matching that at which the observation light sources 30 go off and the alignment light source 82 goes on. This is done in order to avoid any effects by the infrared light beams from the observation light sources 30, 30 during XY-alignment. Since the alignment light source 82 and the observation light sources 30, 30 and turn on and off at faster speed than the speed of conversion to a photoreception signal in the CCD 28, this flashing on and off of the two light sources 82, 30 will not be noticeable on the display screen 110 to which the CCD 28 photoreception signal is output, and to all appearances the two light sources 82, 30 will seem continuously lit.

Next, in S2, the Z-axis actuating mechanism 116 is actuated, and the apparatus optical system 10 is operated so as to advance forward by a prescribed distance in the direction closer to the examined eye E. Then, the location sensing light source 54 emits light, the infrared beam emitted from the location sensing light source 54 is directed in a diagonal direction onto the cornea C of the examined eye E, and the beam reflected from the cornea C is received by the line sensor 44. In the present embodiment in particular, since the beam emitted from the location sensing light source 54 is a beam of infrared light, there is less of a burden on the patient.

The reflected beam is then sensed by the line sensor 44, and a quantity of light distribution signal is sensed by the line sensor 44. The focal location on the corneal epithelium is sensed on the basis of the quantity of light distribution signal; and using the sensed location as a reference, the imaging control circuit 117 will actuate the Z-axis actuating mechanism 116 and advance the apparatus optical system 10 forward by a prescribed distance in the direction closer to the examined eye E. That is, on the basis of the sensor signal from the line sensor 44, the apparatus optical system 10 will be advanced forward so that the focal location in the apparatus optical system 10 is located a prescribed distance away to the rear of the endothelial cells in the cornea C in the Z-direction. Then, a location a prescribed distance to the rear of the corneal epithelium will be designated as the reversal location of the apparatus optical system 10. As will be apparent from the above, in the present embodiment, the Z-direction actuating means includes the Z-axis actuating mechanism 116, while the Z-direction location control means includes the imaging control circuit 117.

Here, the line sensor 44 does not sense the focal location to the corneal endothelium in the Z-direction, that is, it only recognizes that the focal location to the corneal endothelium has been passed; thus, during forward operation of the apparatus optical system 10 on the basis of the line sensor 44 output, Z-direction location control (determination of the reversal location) may be carried out consistently. The distance of movement in the forward direction may be appropriately set to within a range of between 1000 and 1500 $\mu$m forward from the focal location in the Z-direction of the corneal epithelium, for example.

Next, once the apparatus optical system 10 has been positioned at the reversal location, in S3, the Z-axis actuating mechanism 116 is actuated in the opposite direction, and the apparatus optical system 10 is retracted in the direction away from the examined eye E on the Z-axis. During both forward advance and retraction it is possible for the speed of movement of the apparatus optical system 10 to be set differently as appropriate for each movement location.

In S4, at the point in time of retraction by a prescribed distance from the reversal location, the observation light sources 30, 30 are extinguished and the imaging light source 40 begins to emit light. The imaging light source 40 flashes on and off at relatively short intervals, and the XY-alignment of S1 is carried out simultaneously with the timing at which the imaging light source 40 goes off. As noted, this XY-alignment is carried out on the basis of the result of sensing of corneal epithelium reflected light by the alignment sensor 88 at the same timing at which the observation light sources 30 and the alignment light source 82 goes on.

In S5, continuous imaging of the corneal endothelium sensed by the CCD 28 commences at a point in time at which a prescribed level of reflected light from the endothelial cells of the cornea C is sensed by the CCD 28. Continuous imaging is carried out by inputting to the image selection circuit 122 photographing images (pictures) imaged by the CCD 28 at prescribed time intervals (e.g. of 1/30 second). Multiple corneal images taken at different times and locations are thereby input to the image selection circuit 122. Concomitant with continuous imaging, the image selection circuit 122 selectively saves and discards input images, and stores saved ones in the storage device 124.

Possible methods for selectively saving and discarding images in the image selection circuit 122 include, for example, acquiring luminance values of pixels on several horizontal lines in images acquired by the CCD 28, and using these values to determine the contrast of the image, in order to selectively save only images that effectively afford images of the corneal endothelial cells.

After retraction by a sufficient distance inclusive of the focal point of the corneal endothelial cells (i.e. a distance beyond the focal point), in S6, the retraction operation will come to a halt, the imaging light source 40 will extinguish, and imaging or photography will terminate.

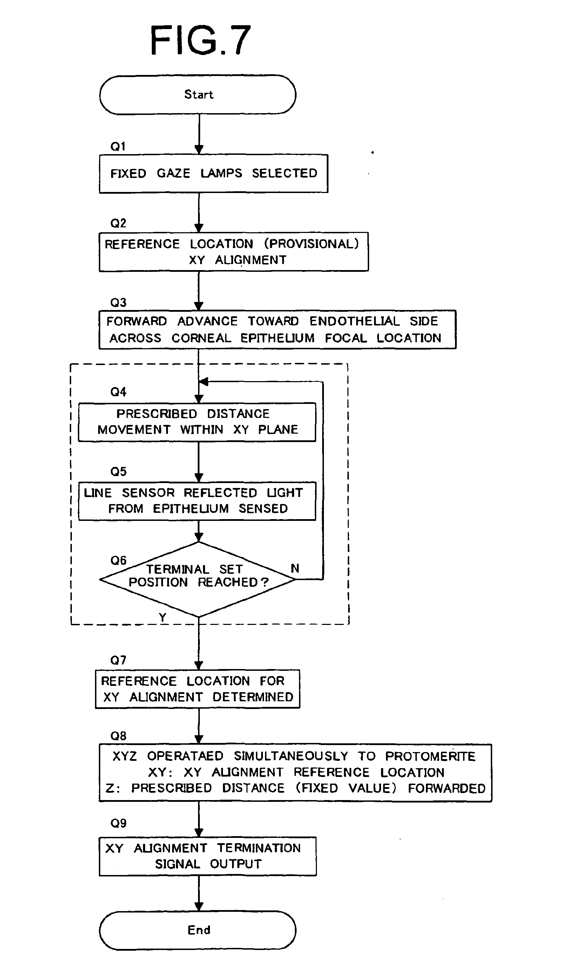

The description now turns to the case of photographing the endothelium in a peripheral part of the cornea of the examined eye E. The operation of photographing the endothelium in a peripheral part of the cornea may be carried out with only S1 and S2 differing to any substantial extent as compared to photographing the endothelium in the center part of the cornea; accordingly, only steps corresponding to the processes in the operations of S 1 and S2 will be described here, with reference to FIG. 7.

Specifically, after initiating endothelial imaging in a peripheral part of the cornea, first, in Step Q1, any one of the peripheral fixed gaze lamps 89 is selected and lit. The technician then instructs the patient to fix his or her gaze on the lit fixed gaze lamp 89. By so doing, the visual axis of the examined eye E will be fixed in a state inclined towards a specific direction corresponding to the one selected peripheral fixed gaze lamp 89, and inclined by a specific angle with respect to the visual axis: O1 of the frontal vision state specified by the center fixed gaze target optical system 64.

Next, in Step Q2, a process substantially identical to the XY-alignment in S1 described earlier is carried out to execute (provisional) XY-alignment. Specifically, first, the alignment light 126 directed onto the examined eye E from the alignment light source 82 is reflected by the corneal epithelium of the examined eye E. Then the XY location of straight reflected light from the corneal epithelium is sensed by the alignment sensor 88 and is input to the XY-alignment sensor circuit 118. On the basis of this XY location information, the optical axis O1 of the observation optical system 12 is aligned with the optical axis of straight reflected light from the corneal epithelium of the examined eye E in order to execute (provisional) XY-alignment. The expression (provisional) is used here because, as is well known, due to the different curvature radii of the corneal epithelium and the corneal endothelium, where the visual axis of the examined eye is inclined, the corneal epithelium will be orthogonal to the optical axis O1 at its point of intersection with the optical axis O1, and will give rise to frontal reflection on the same optical axis O1 as the light from the XY-alignment light source 82 projected on the optical axis O1. Whereas the corneal endothelium targeted for photographing will not be orthogonal to the optical axis O1 at its point of intersection with the optical axis O1 and thus will not give rise to specular reflection on the same optical axis O1 as the light from the XY-alignment light source 82 projected on the optical axis O1. That is, the aforementioned expression (provisional) XY-alignment is used because alignment is primarily with respect to the axis of straight reflected light from the corneal epithelium (peripheral part) and as such cannot be considered as correct XY-alignment with respect to the axis reflected light from the corneal endothelium which is actually targeted for photographing.

Next, in Step Q3, a forward advance process is carried out. The forward advance operation of Step S2 discussed earlier will be completed at some stage after the forward advance process has been initiated in Step Q3 and before XY-alignment is completed in Step Q9 discussed later. Specifically, first, in Step Q3, the apparatus optical system 10 will be advanced forward in the direction closer to the examined eye E by the Z-axis actuating mechanism 116. The reflected light of the4 infrared beam emitted by the location sensing light source 54 will be received by the line sensor 44. This information will be input to the Z alignment sensor circuit 120, and the location of the imaging optical system 20 of the apparatus optical system 10 in the Z-direction will be sensed. In the present embodiment in particular, location sensing is carried out on the basis of the output pattern of the line sensor 44.

Location sensing on the basis of the line sensor 44 output is known art, having been disclosed inter alia in JP-B-7-121255, and will not be discussed in detail here. In the present embodiment in particular, endothelial focusing for the purpose of endothelial imaging, which requires a high degree of accuracy, is not carried out on the basis of the output pattern of the line sensor 44. As will be discussed later, continuous imaging rendered endothelial focusing on the basis of sensor results of the line sensor 44 unnecessary.

Moreover, in the present embodiment, the Z-direction location of the apparatus optical system 10 is adjusted by the Z-axis actuating mechanism 116 on the basis of sensor results of the line sensor 44. With respect to this point, in the present embodiment, the focal location of the corneal epithelial cells is sensed by the line sensor 44, and the Z-direction location of the apparatus optical system 10 is set such that the focal location of the observation optical system 12 in the apparatus optical system 10 is situated past the focal location for the corneal epithelial cells of the examined eye E, for example, at a location 500 $\mu$m closer towards the corneal endothelium side from the focal location of the corneal epithelial cells. While the specific distance setting bringing the apparatus optical system 10 past the point of focus on the corneal epithelial cells of the examined eye E and closer to the corneal endothelium side can be fixed in advance from anatomical statistical data as discussed previously for example, alternatively, from the sensor results of the line sensor 44, the distance setting can be made with reference to the focal location of the corneal endothelium, making it possible to eliminate the problem of individual differences.

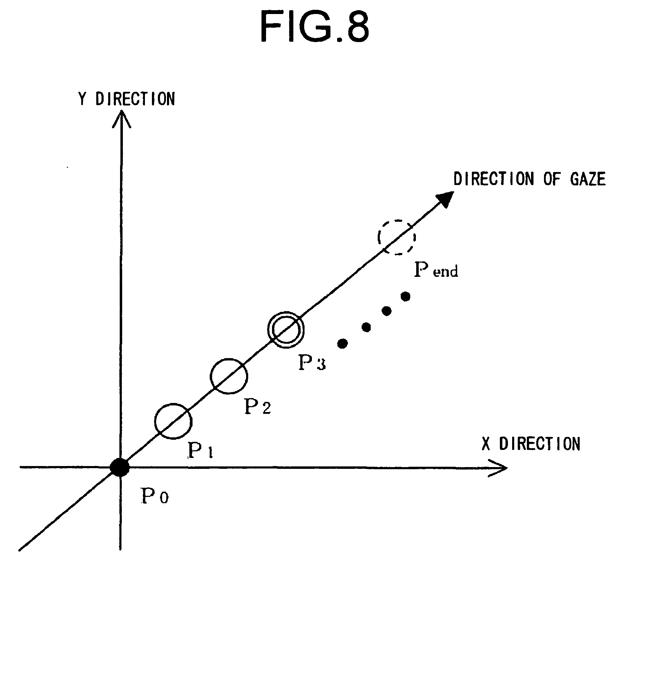

After setting the location, on the basis of a loop operation of Steps Q4 to Q6, starting from a (provisional) XY-alignment location denoted as P0, the apparatus optical system 10 will be moved in increments of prescribed distance towards a specific XY-direction which corresponds to the direction of gaze of the patient, to locations denoted as P1, P2, P3 ... in the manner shown in FIG. 8. At each of these locations reflected light of the infrared beam emitted by the location sensing light source 54 will be received by the line sensor 44, acquiring multiple data points of quantity of light distribution information by way of corneal endothelium optical information for XY-alignment purposes. Subsequently, at the point in time that a final preset endpoint Pend is reached, acquisition of quantity of light distribution information will terminate.

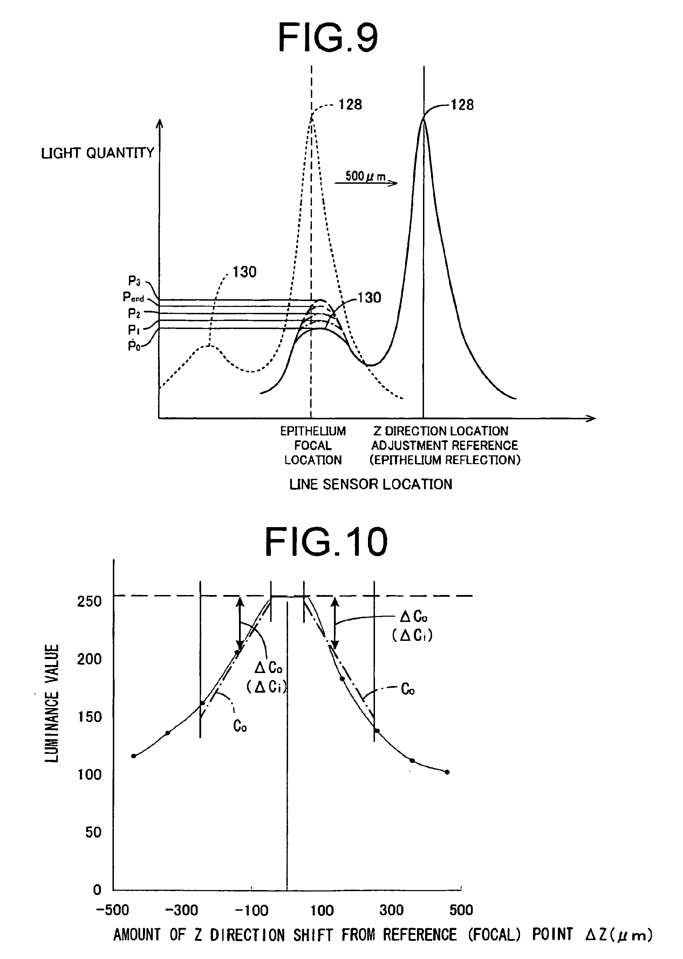

FIG. 9 is an illustration depicting quantity of light distribution sensed by the line sensor 44. In FIG. 9, a first peak 128 associated with the highest quantity of light indicates reflected light from the corneal epithelium. A second peak 130 associated with the next-highest quantity of light indicates reflected light from the corneal endothelium. In actual practice sensor values will reach saturation in the section indicating reflected light from the corneal epithelium. However, in FIG. 9 a mountain-shaped peak is shown for convenience.

The graph shown by the broken line on the left side in FIG. 9 gives sensor results of the line sensor 44 at a point at which the apparatus optical system 10 has been set to the corneal epithelium focal location in the Z-direction, which is used for alignment in the Z-direction in the aforementioned Step Q3. In the aforementioned Step Q3, by advancing the system by an additional 500 $\mu$m inward from the corneal epithelium focal location in the Z-direction, the sensor results of the line sensor 44 will change to a graph like that shown by the solid line on the right side in FIG. 9. As will be apparent from a comparison of the left and right graphs, in the present embodiment, by moving the apparatus optical system 10 past the point of focus on the corneal epithelial cells of the examined eye E and closer towards the corneal endothelial side, the quantity of light reflected by the corneal endothelium, used in determination of XY-alignment reference location to be discussed later, can be better sensed.

With alignment in the Z-direction having been performed in the aforementioned Step Q3, by moving the apparatus optical system 10 in the XY-direction to each of the points P1, P2, P3 ... shown in FIG. 8, the sensed value of quantity of light reflected by the corneal endothelium, represented as the second peak 130 in FIG. 9, will change.

The quantity of reflected light from the endothelium in the quantity of light distribution information acquired by the line sensor 44 at each of the P1, P2, P3 ... in accordance with Step Q5 may be appropriately subjected to correction of sensor values as described below. Specifically, in the process of moving the apparatus optical system 10 in the Z-direction in accordance with the loop operation of Steps Q4 to Q6, the relative location of the apparatus optical system 10 in the Z-direction with respect to the corneal endothelium may vary due to factors such as curvature of the cornea or movement by the patient. If such deviation of relative location of the apparatus optical system 10 in the Z-direction with respect to the corneal endothelium should occur, the sensed quantity of light reflected from the corneal endothelium will decrease depending on the extent of deviation of the apparatus optical system 10 from the corneal endothelial focal point (the reference location aligned in Step Q3), resulting in drop in sensing accuracy of the quantity of light reflected from the corneal endothelium. Accordingly, correction such as the following may be advantageously carried out in order to avoid measurement errors caused by such unwanted Z-direction displacement.

By way of one correction (first correction), a strategy of direct correction of values for quantity of light reflected from the corneal endothelium sensed by the line sensor 44 may be employed.

Specifically, first, on the basis of the photoreception characteristics of corneal epithelial reflected light by the line sensor 44 used as shown in FIG. 10, there may be derived a correlation relationship between Z-direction shift of the peak location of corneal epithelial reflected light and the amount of change in sensed quantity of light. In the specific example shown in FIG. 10, the peak of corneal epithelial reflected light reaches saturation at a luminance value of approximately 250 in an area extending approximately 100 $\mu$m in the Z-direction forward and back the corneal epithelium focal location. To either side of this saturation area, correlative change is observed through areas each of approximately 200 $\mu$m forward and back in the Z-direction. This correlation relationship can be approximated by the linear expressions given by Equations 1 and 2 below.

$$Co = \Delta Z/2 + 280 \ (-200 \leq \Delta Z \leq -50) \qquad \dots \text{(Equation 1)}$$

$$Co = -\Delta Z/2 + 280 \ (-50 \leq \Delta Z \leq 200) \qquad \dots \text{(Equation 2)}$$

In Equations 1 and 2, $\Delta Z$ denotes Z-direction deviation ($\mu$m), and Co denotes a luminance value which is the value of corneal epithelial reflected light sensed by the line sensor.

Next, the line sensor 44 derives a correlation relationship between change in value of sensed corneal epithelial reflected light (luminance value) and change in value of sensed corneal endothelial reflected light (luminance value), occurring respectively in association with displacement in the Z-direction. In a specific example, the amount of change in value of sensed corneal epithelial reflected light (luminance value) and the amount of change in value of sensed corneal endothelial reflected light (luminance value) are approximately the same. Where this is true, in consideration of the aforementioned (Equation) 1 and (Equation) 2, the calculated change in value of sensed corneal endothelial reflected light, i.e. the correction level Ci, can be expressed by Equations 3 and 4 below.

$$\Delta Ci = \Delta Z/2 - 25 \ (-200 \leq \Delta Z \leq -50) \qquad \dots \text{(Equation 3)}$$

$$\Delta Ci = \Delta Z/2 - 25 \ (50 \leq \Delta Z \leq 200) \dots \text{(Equation 4)}$$

Thus, by adding a correction level: $\Delta Ci$ derived with Equation 3 or 4 to the sensed value of corneal epithelial reflected light (luminance value) actually sensed by the line sensor 4, there can be obtained a corrected value of corneal epithelial reflected light in which error caused by Z-direction displacement has been corrected.

In the example described above, the value of sensed corneal endothelial reflected light reaches saturation in a range in which the amount of Z-direction deviation $\Delta Z$ from the peak location is represented by the following expression, and thus correction with Equation 3 or 4 will not be performed in this range.

$$-50 \ \mu m \leq \Delta Z \leq 50 \ \mu m$$

It would not be mistaken to posit that, even away from the optical axis of frontal reflection from the corneal epithelium, saturation of sensed values of corneal epithelial reflected light by the line sensor 44 will be manifested, at least at any of the measurement locations P1, P2, P3 ... shown in FIG. 8.

As will be apparent from the preceding description, in the first correction according to the present embodiment, the computing means of the computer which executes the aforementioned (Equation 3) and (Equation 4) using the level of corneal endothelial reflected light as the corneal endothelium optical information acquired by the line sensor 44 and the level of corneal epithelial reflected light as the corneal epithelium optical information to calculate the correction level: $\Delta Ci$ for corneal endothelial reflected light constitutes the "optical information correction level computing means". The computing means of the computer which adds the calculated correction level: $\Delta Ci$ to the level of corneal endothelial reflected light acquired by the line sensor 44 constitutes the "optical information correction executing means." As this computer, the computer which uses the location control means for the optical systems in the XY-direction and Z-direction can be employed.

By way of the other correction (second correction), a strategy for location correction (control) of the Z-direction location of the corneal endothelium sensed by the line sensor 44 so as to be generally fixed with respect to the apparatus optical system 10 inclusive of the line sensor 44 as the photoreceptor element could also be employed.

Specifically, in the same manner as in the first correction described above, there may be derived a correlation relationship between Z-direction shift of the peak location of corneal epithelial reflected light and the amount of change in sensed quantity of light, on the basis of the photoreception characteristics of corneal epithelial reflected light by the line sensor 44 used as shown in FIG. 10, to derive the aforementioned Equations 1 and 2.

Next, for sensor values of corneal epithelial reflected light (luminance value) actually sensed by the line sensor 44, it is then ascertained whether the sensor values equal a saturation value which has been pre-calculated. If a value happens to be a saturation value, it will be decided that substantially no Z-direction displacement has occurred, and the value of corneal epithelial reflected light sensed by the line sensor 44 will be employed as-is. If on the other hand the value is not a saturation value, Z-direction deviation: $\Delta Z$ will be calculated using the aforementioned (Equation 1) or (Equation 2). Location correction will then be performed through relative displacement of the apparatus optical system 10 with respect to the examined eye E in the Z-direction by the equivalent of the calculated Z-direction deviation: $\Delta Z$. During this process, corneal endothelial reflected light will again be sensed by the line sensor 44, and the sensed value (luminance value) will be adopted as the sensor value of corneal endothelial reflected light at the XY-direction location in question.

During location correction of the apparatus optical system 10 in the Z-direction on the basis of the amount of deviation: $\Delta Z$, it will be difficult to calculate, on the basis of the aforementioned (Equation 1) or (Equation 2) only, which direction location correction should be carried out in the Z-direction (i.e. whether in the direction closer to or in the direction away from the examined eye E). However, it is possible to estimate the correction direction statistically from the diagonal direction of the cornea. This can also be addressed by after location correction in the Z-direction, by re-checking for the presence of Z-direction displacement, from the quantity of light of corneal epithelial reflected light sensed by the line sensor 44.

As will be apparent from the preceding description, in the second correction according to the present embodiment, the computing means of the computer which calculates the Z-direction correction level: $\Delta Z$ with the aforementioned (Equation 1) and (Equation 2) using the level of corneal endothelial reflected light as the corneal endothelium optical information acquired by the line sensor 44 and the level of corneal epithelial reflected light as the corneal epithelium optical information constitutes the " Z-direction change computing means". The control system of the Z-direction actuating means which performs location correction of the apparatus optical system 10 with respect to the examined eye E in the Z-direction on the basis of the calculated correction level: $\Delta Z$ constitutes the "Z-direction location correction executing means."

A yet another strategy for correction (third correction) provides an example of strategy for location correction (control) of the Z-direction location of the corneal endothelium sensed by the line sensor 44 so as to be generally fixed with respect to the apparatus optical system 10 inclusive of the line sensor 44. Namely, it is possible to employ a correction method wherein the peak location of corneal epithelial reflected light is sensed in the line sensor 44, and location correction is carried out through relative displacement of the apparatus optical system 10 with respect to the examined eye E in the Z-direction by a distance equivalent to the amount of deviation of the peak location from a fixed location, so that the sensed peak location is brought to this pre-established fixed location on the line sensor 44.

Specifically, the peak location of corneal endothelial reflected light on the line sensor 44 (the peak location of corneal epithelial reflected light) corresponds to relative position of the apparatus optical system 10 to the examined eye E (more specifically the corneal epithelium) in the Z-direction. In the examined eye E, the distance separating the corneal epithelium and the corneal endothelium in the Z-direction is not large enough to pose a problem during acquisition of corneal endothelium optical information for XY-alignment purposes. Accordingly, through relative location adjustment of the apparatus optical system 10 with respect to the examined eye E in the Z-direction so that the peak location of corneal epithelial reflected light is brought to a pre-established fixed location on the line sensor, it will be possible to carry out correction which minimizes Z-direction displacement of the corneal endothelium.

After carrying out Z-direction location correction in this way, it will be possible to sense endothelial reflected light and use the sensed value (luminance value) as the sensor value of corneal endothelial reflected light at the XY-direction location in question.

Correlation relationships between the amount and direction of shift of the peak location of corneal epithelial reflected light, and the corresponding Z-direction correction level of the apparatus optical system 10 with respect to the examined eye E, can be derived in advance through experimentation, testing, computations, or the like. In this third correction method as well, in the event that the sensor value of corneal epithelial reflected light by the line sensor 44 reaches saturation in a prescribed area, the center point of the saturation area may be designated as the peak location.

Where a method like this third correction method is employed, it is possible to deal easily with relatively large Z-direction displacement as compared with the first correction method or second correction method. Consequently, instances in which the amount of Z-direction displacement is relatively large can be dealt with using the third correction method. If needed, once the amount of Z-direction displacement has become smaller, the first correction method or second correction method can be additionally employed in a subsequent step.

In this third correction, the computing means of the computer which, using peak location information on the line sensor 44 of sensor values of corneal endothelial reflected light as corneal endothelium optical information acquired by the line sensor 44, calculates the amount of deviation of the peak location from a pre-established specific location on the line sensor 44, and which from the resultant deviation on the line sensor 44 calculates an amount of Z-direction deviation (correction level) $\Delta Z'$ of the apparatus optical system 10 with respect to the examined eye E, constitutes the "Z-direction change computing means". The control system of the Z-direction actuating means which performs location correction of the apparatus optical system 10 with respect to the examined eye E in the Z-direction on the basis of the calculated correction level: $\Delta Z'$ constitutes the "Z-direction location correction executing means."

During loop operation of Steps Q4 through Q6, for reasons relating to the structure of the eye, the specific XY-direction in which the apparatus optical system 10 is moved can be set, for example, to a direction which is line-symmetrical in relation to the optical axis: O1 of frontal reflected light from the corneal epithelium specified by the center fixed gaze target optical system 64, with respect to the lit fixed gaze lamp 89 situated in the direction of the visual axis of the examined eye E (i.e. the direction opposite the diagonal direction of the visual axis of the examined eye E).

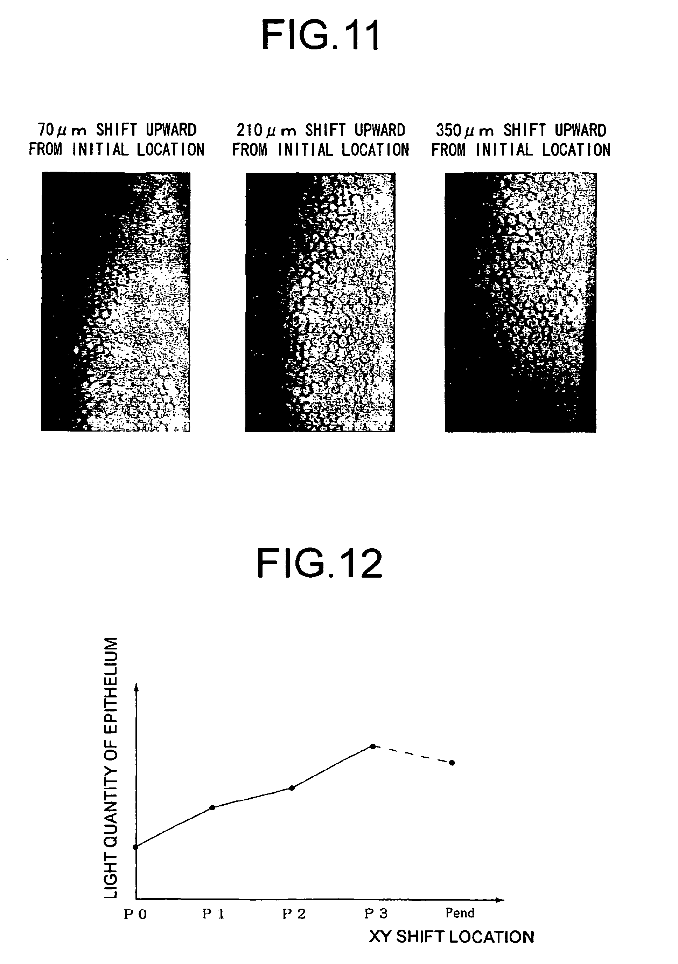

Images of the corneal endothelium photographed by the CCD 28 at various locations of movement in the XY-direction on the opposite side from the diagonal direction of the visual axis of the examined eye are shown for reference in FIG. 11. These pictures of corneal endothelium were photographed with gaze fixed in the 12 o-clock direction in FIG. 2, and with the apparatus optical system 10 moving in the corresponding direction; from these pictures of corneal endothelium as well, it will be appreciated that different corneal endothelium images obtained depending on the extent of movement in the XY-direction (i.e. the amount of deviation with respect to the frontal reflection axis of the corneal endothelium).

The location of Pend, which is the point at which acquisition of the quantity of corneal endothelial reflected light terminates, can be established in consideration of the angle of slope of the examined eye, on the basis of the structure of the eye or anatomical statistical data. Since the purpose of the loop process of Steps Q4 through Q6 is to calculate the frontal reflection axis of the corneal endothelium, if the frontal reflection axis of the corneal endothelium can be estimated on the basis of, for example, the fact that the quantity of corneal endothelial reflected light has reached a predetermined reference value, it will be acceptable to terminate sensing of the quantity of reflected light by the line sensor 44 at that location before the Pend location is reached.

In the subsequent Step Q7, on the basis of comparison with the quantity of corneal endothelial reflected light indicated by the line sensor 44 sensor results which were acquired in the process according to Steps Q4 through Q6, a reference location for XY-alignment purposes is determined. In the present embodiment, in a quantity of light distribution like that depicted in FIG. 9 acquired by the line sensor 44, first, using as a reference the first peak 128 (epithelial reflection) which shows the largest peak, a sensor frame of between 300 $\mu$m and 800 $\mu$m in width for quantity of corneal endothelial reflected light is defined. This frame corresponds to an area in which corneal endothelial reflected light is sensed and situated in proximity to the second peak 130, i.e. in proximity to a location shifted approximately 500 $\mu$m inwards towards the corneal endothelium side from the first peak 128. In order to eliminate sensor error, in the sensor frame [values] below a given value will be discarded, and the integration value of valid values which are greater than the specified value will be computed in order to calculate the quantity of reflected light from the corneal endothelium. Here, comparison of computed values of corneal endothelial reflected light at the aforementioned points P1, P2, P3 ... Pend will give a result as shown in FIG. 12. These values are compared with one another, and the point associated with the maximum quantity of corneal endothelial reflected light (e.g. P3 in FIG. 12) will be designated as the XY-alignment reference location. As the method for comparing the quantity of corneal endothelial reflected light at each of the points P1, P2, P3 ... Pend, besides the method of calculating an integration value within a given sensor frame as described above, it would be possible to instead compare values with one another using the maximum value of quantity of light sensed within the sensor frame. In this case in particular, it will be preferable to concomitantly carry out a correction process of corneal endothelial reflected light quantity on the basis of the amount of deviation $\Delta Z$ of the apparatus optical system 10 in the Z-direction.

In the present embodiment, in consideration of relative deviation of the axis of frontal reflection from the corneal epithelium (orthogonal axis to the corneal epithelium) and the axis of frontal reflection from the corneal endothelium (orthogonal axis to the corneal endothelium), the apparatus optical system 10 is moved in a specific XY-direction from a (provisional) XY-alignment location which is the location of the axis of frontal reflection from the corneal epithelium. Using the quantity of reflected light from the corneal endothelium indicated by sensor results of the line sensor 44 acquired at each point after movement, the location of the axis of frontal reflection from the corneal endothelium is estimated in order to determine a reference location for XY-alignment purposes, as the specific location in the XY-direction.

As will be apparent from the preceding description, in the present embodiment, the line sensor 44 is included in the constitution of the XY-alignment optical information acquisition control means. Step Q7 is included in the XY-alignment reference location determining means.

Next, in Step Q8, a further forward advance operation to a reversal/retraction operation start location corresponding to the aforementioned Steps S2 and S3 is carried out. Then, in Step Q9 an XY-alignment termination signal is output, whereupon the system returns to the aforementioned Step S3 and executes the process beginning with Step S3.

During this process, simultaneous with forward advance in the Z-direction, the apparatus optical system 10 which was set at the (provisional) XY-alignment location in the XY-direction in the aforementioned Step Q2 will now be moved in the XY-direction and guided to the alignment reference location that was determined in the aforementioned Step Q7. By carrying out movement in the XY-direction to the XY-alignment reference location simultaneously with alignment in the Z-direction in this way, the time required for corneal endothelium imaging can be shortened, and the burden on the patient can be reduced.

Subsequently, the objective corneal endothelium can be photographed by executing the process beginning with Step S3 from Step Q9. During imaging of the corneal endothelium as well, XY-alignment by the alignment sensor 88 is carried out as appropriate. Specifically, in generally the same manner as for imaging of the corneal endothelium in the aforementioned Steps S4 and S5, the location of frontal reflected light by the corneal epithelium of the beam from the alignment light source 82 is sensed by the alignment sensor 88 during times that the flashing imaging light source 40 is off. Correction of XY-alignment is then executed by the imaging control circuit 117 on the basis of the frontal reflection sensed location information, and the alignment reference location information that was determined in Step Q7.

That is, XY-alignment is carried out in such a way that the imaging optical system 20 will always be positioned at an XY-alignment reference location which is shifted towards a specific XY-direction by the amount of deviation computed in Step Q7, with respect to the location at which frontal reflected light from the corneal epithelium is sensed ((provisional) XY-alignment location). Thus, in the present embodiment, the constitution of the corneal endothelium photographing image control means includes the imaging control circuit 117 and the aforementioned Steps S3 through S5. Also, as mentioned above, the XY-alignment reference location verification means may be constituted by the "XY-alignment correction control means" that is composed of various constitutions whereby correction of XY-alignment is executed using the intervals during continuous imaging of the corneal endothelium as described previously.

By executing corneal endothelium imaging utilizing XY-alignment reference location determining means, and corneal endothelium corneal endothelium photographing image control means which utilizes a reference location determined thereby, it will be possible on the basis of a sensor signal from the corneal epithelium reflected light XY-alignment photoreceptor system to align the imaging optical system 20 at a location at which the optical axis O1 is orthogonal to the corneal endothelium, even where the visual axis of the examined eye E is inclined away from the optical axis O1 of frontal reflection.

Accordingly, even with an imaging operation identical to that during frontal reflection in Steps S3 through S6, that is, without any special processes or operations, it will be possible to automatically obtain clear endothelial images of consistently high accuracy.

In particular, it should be noted that since XY-alignment can be carried out on the basis of individual patient information rather than simple statistical values, XY-alignment can be carried out with high accuracy on an individual basis. Additionally, since XY-alignment can be carried out directly using images of the corneal endothelium rather than estimating from images of the corneal epithelium, it is possible for the desired images of the corneal endothelium to be obtained with higher accuracy on a consistent basis.

Next, a cornea imaging apparatus will be described by way of a second embodiment of the present invention. In the first embodiment described previously, in Step Q5, quantity of reflected light from the corneal endothelium acquired by the line sensor 44 is used as the corneal endothelium optical information for XY-alignment purposes. In the present embodiment, however, an corneal endothelium image for XY-alignment purposes photographed with the CCD 28 is used as the corneal endothelium optical information for XY-alignment purposes for acquisition in Step Q5, and in Step Q7 an XY-alignment reference location is determined using optical information of this XY-alignment corneal endothelium image. Moreover, in the present embodiment, the configuration is similar to that in the first embodiment except for this Step Q5 and Step Q7, and thus only processes which correspond to Steps Q5 through Q7 in the preceding first embodiment will be discussed below.

Specifically, in Step Q5 of the loop operation of Steps Q4 to Q6 in the previous first embodiment, starting from a (provisional) XY-alignment location denoted as P0 in FIG. 8, the apparatus optical system 10 is moved in increments of prescribed distance towards a specific XY-direction which corresponds to the direction of gaze of the patient to points P1, P2, P3 .... However, in the present embodiment, rather than acquiring a quantity of light distribution information with the line sensor 44 in manner of the first embodiment, instead, multiple images of the corneal endothelium for XY-alignment purposes are photographed by the CCD 28. Then, in the same manner as in the first embodiment, corneal endothelium imaging for XY-alignment purposes terminates at a point in time that a final preset endpoint Pend is reached.

Here, photographing of images of the corneal endothelium for XY-alignment in Step Q5 will be carried out by extinguishing the observation light sources 30, 30 and lighting the imaging light source 40, and photographing images of the corneal endothelium for XY-alignment with the CCD 28 of the imaging optical system 20. At this time, the quantity of light emitted from the imaging light source 40 in the imaging illumination optical system 14 may be smaller than the quantity of light during imaging of the corneal endothelium in the aforementioned Step S4. This is because the ultimate purpose is to check XY-alignment, not to photograph an image of the corneal endothelium for observation purposes. The burden on the patient can be ameliorated to some extend by dropping the quantity of light during imaging in this way.

Moreover, during the loop operation of Steps Q4 to Q6, in the same manner as in the first embodiment, while it is possible to set the specific XY-direction for moving the apparatus optical system 10 to a direction which is line-symmetrical in relation to the optical axis: O1 of frontal reflected light from the corneal epithelium specified by the center fixed gaze target optical system 64, with respect to the lit fixed gaze lamp 89 situated in the direction of the visual axis of the examined eye E (i.e. the direction opposite the diagonal direction of the visual axis of the examined eye E), is would also be possible to use another method such as the following, for example.

Specifically, by way of another method for specifying a specific XY-direction for moving the apparatus optical system 10, would be possible, for example, with the visual axis of the examined eye E inclined by an illuminated fixed gaze lamp 89, to photograph with the imaging optical system 20 an image of the corneal epithelium or an image of the corneal endothelium on the optical axis O1 of frontal reflected light from the corneal epithelium; and using optical information of this corneal epithelium image or corneal endothelium image, to decide an XY-direction in which to move the apparatus optical system 10. The inventors have found that, with the visual axis of the examined eye E inclined, differences in contrast and gray spots corresponding to the direction where the axis of frontal reflected light from the corneal endothelium is located, thought to be caused inter alia by differences in relative curvature of the corneal epithelium and the corneal endothelium, are observed in corneal epithelium images or corneal endothelium images photographed at the (provisional) XY-alignment location. Consequently, where a correlation relationship between a specific XY-direction of movement of the apparatus optical system 10 (i.e. direction of relative deviation of the axis of frontal reflected light from the corneal endothelium with respect to the axis of frontal reflected light from the corneal epithelium), and optical information such as gray spots in a corneal epithelium image or corneal endothelium image that was photographed on the axis of frontal reflected light from the corneal epithelium has been calculated in advance through experimentation or the like, it will be possible to determine a specific XY-direction for moving the apparatus optical system 10 from the optical information of a corneal epithelium image or corneal endothelium image photographed at the (provisional) XY-alignment location obtained thereby.

As in the preceding first embodiment, the location of Pend which is the point at which imaging of the corneal endothelium terminates can be set in consideration of the angle of incline of the examined eye, on the basis of the structure of the human eye, anatomical statistics, and so on. Moreover, in the present embodiment, it is possible to specify the axis of frontal reflected light from the corneal endothelium from optical information of a picture of the corneal endothelium obtained thereby. Specifically, the axis of frontal reflected light from the corneal endothelium can be estimated based on the fact that contrast and lighting of a photographed image are uniform overall, for example. Consequently, since the purpose of the loop process of Steps Q4 through Q6 is to derive the axis of frontal reflected light from the corneal endothelium, if the axis of frontal reflected light from the corneal endothelium can be estimated on the basis of optical information such as contrast in a photographed image, corneal endothelium imaging may be terminated at that location before the Pend location is reached.

Then, in the subsequent Step Q7, an XY-alignment reference location is computed on the basis of optical information in the corneal endothelium image obtained by the process according to Steps Q4 through Q6. In view of the fact noted previously that contrast and lighting of the photographed image are uniform overall, the computation could involve estimation of the axis of frontal reflected light from the corneal endothelium etc. Of course, it would be possible for the technician to make this decision by sight, or to have a computer process the digitized information.

It will be possible to advantageously determine an XY-alignment reference location through acquisition of corneal endothelium optical information through photography of corneal endothelium images for XY-alignment in the above manner. As will be apparent from the preceding description, in the present embodiment, the constitution of the XY-alignment optical information acquisition control means includes the imaging illumination optical system 14 and the imaging optical system 20.

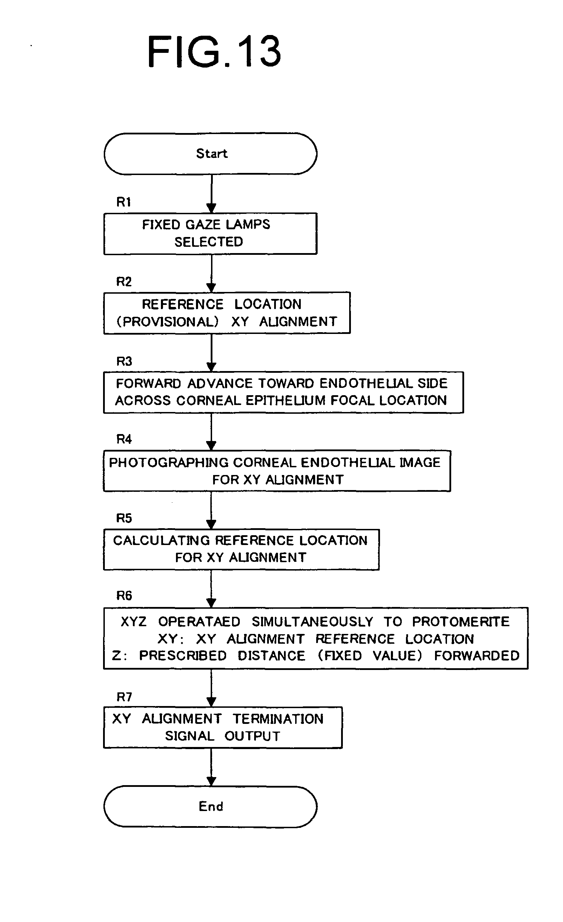

Next, a cornea imaging apparatus will be described by way of a third embodiment of the present invention. The embodiment hereinbelow shows an another specific example of determination of XY-alignment reference location. Since only the operation shown by Steps Q1 through Q9 of FIG. 7 in the preceding first embodiment is different, the description here will make reference to the process diagram in FIG. 13, which corresponds to FIG. 7 in the preceding first embodiment.

First, in Steps R1 through R3, in the same way as in the aforementioned Steps Q1 through Q3, the apparatus optical system 10 is aligned in the XY-direction to a (provisional) XY-alignment location, and in the Z-direction to a location past the corneal epithelium focal location and closer to the corneal endothelium side by a prescribed amount.

After setting the location, in Step R4, in the same manner as the operation in Step Q5 of the second embodiment, the observation light sources 30, 30 will be extinguished, the imaging light source 40 will be lit, and a corneal endothelium image for XY-alignment purposes will be photographed by the CCD 28 of imaging optical system 20.

In the present embodiment, as in the previous second embodiment, the constitution of the XY-alignment optical information acquisition control means includes the imaging illumination optical system 14 and the imaging optical system 20. In the present embodiment, photography of the corneal endothelium image for XY-alignment purposes is carried out on the axis of frontal reflected light from the corneal epithelium, subsequent to (provisional) XY-alignment in Step R2.

Next, in Step R5, an XY-alignment reference location is determined on the basis of the corneal endothelium image for XY-alignment purposes that was obtained by the CCD 28. In the present embodiment, by estimating the curvature radius of the corneal endothelium from optical information of the corneal endothelium image for XY-alignment, it is in turn estimated how far away the location of the optical axis of frontal reflected light from the corneal endothelium is from the optical axis of frontal reflected light from the corneal epithelium, and the XY-alignment reference location is determined thereby.

In relation to the corneal endothelium image for XY-alignment purposes obtained in the aforementioned Step R4, the inventors have found that even where a patient's gaze is fixed in a given direction at a given angle, there are individual differences among patients. This is because, even under identical photographing conditions, optical information in corneal endothelium images for XY-alignment purposes (e.g. contrast differences or the extent or severity of gray spots) will differ for each individual patient. Research conducted by the inventors led to the discovery that such individual differences in optical information are due primarily to differences in curvature radius of the corneal endothelium among individual patients. Specifically, the curvature radius of the corneal endothelium can be estimated from these variations in contrast or extent of localized gray spots in corneal endothelium images for XY-alignment purposes; and on the basis of the estimated value, deviation of the optical axis of frontal reflected light from the corneal endothelium with respect to the optical axis of frontal reflected light from the corneal epithelium of the examined eye can be calculated.

Accordingly, in the present embodiment, data are acquired in advance of statistical values representing correlation between deviation of the optical axis of frontal reflected light from the corneal endothelium with respect to the optical axis of frontal reflected light from the corneal epithelium in a large number of examined eyes, and optical information in corneal endothelium images photographed on the optical axis of frontal reflected light from the corneal epithelium in the examined eyes. Thus, it will be possible on the basis of this correlation data to calculate the XY-alignment reference location in Step R5 from optical information of the corneal endothelium image for XY-alignment obtained in Step R4.

Where the XY-alignment reflected light has been estimated in this way, it will be possible to subsequently verify the validity of the XY-alignment reference location, for example, by moving the imaging illumination optical system 14 and the imaging optical system 20 in the XY-direction to the calculated XY-alignment reference location, photographing an image of the corneal endothelium for XY-alignment purposes, and verifying validity on the basis of optical information of the image of the corneal endothelium for XY-alignment purposes obtained thereby. Verification may be performed by the technician by checking the monitor directly by sight; an operation by a computer is possible as well, in consideration of the overall distribution of contrast, luminance level, and so on.

In the event that verification in this manner leads to a decision that the XY-alignment reference location calculated in Step R5 is erroneous, the system will return to from Step R4 to the preceding steps, and repeat photographing of the image of the corneal endothelium for XY-alignment purposes. During this process, in order to prevent the same error from recurring, the image of the corneal endothelium for XY-alignment may be photographed at a location displaced by a prescribed distance in the XY-direction from the axis of reflected light from the corneal epithelium, for example.

Subsequently, in Steps R6 and R7, processes corresponding to Steps Q8 and Q9 in the previous first embodiment will be executed, and the objective corneal endothelium imaging will be executed starting from Step S3.

The third embodiment of course affords advantages similar to the first and second embodiments. Additionally, since it is possible to achieve a reduction in the number of photographs of the corneal endothelium for XY-alignment purposes as compared with the second embodiment, it will be possible to reduce the burden on the patient caused by the imaging light source 40 emitted during imaging of the corneal endothelium for XY-alignment purposes.

While the present invention has been described in detail herein in terms of certain preferred embodiments, the present invention is in no wise limited thereby, and various modifications, adjustments, and improvements thereto will be apparent to the practitioner of the art; and such embodiments shall of course be considered to fall within the scope of the present invention insofar as they do not depart from the spirit of the invention.

For example, the apparatus optical system 10 described hereinabove is merely exemplary, and the configuration and location of placement of the lenses and slits making up the optical systems are not limited to the configurations described hereinabove. For example, whereas in the preceding embodiments, the cold mirror 27 is disposed on the optical axis of the observation optical system 12, in place of the cold mirror 27, it would be possible for example to position a mirror which gives rise to total reflection of the received beam at a location which is away from the optical axis O1, and which reflects the beam from the imaging light source 40 onto the CCD 28.

It is not always necessary for the observation light sources 30, 30 to be infrared light sources, and visible light sources may be used instead. Alternatively, a mirror which gives rise to total reflection of the received beam could be positioned moveably on the optical axis of the observation optical system 12, and selectively switched between a state of directing the beam from the imaging optical system 20 on the CCD 28 while blocking the beam from the observation optical system 12, and a state of being moved to a location away from the optical axis O1 of the observation optical system 12 so that the beam from the observation optical system 12 is directed onto the CCD 28. Also, the locations of the location sensing light source 54 and the line sensor 44 may be switched.

The line sensor 44 in the second and third embodiments is not always necessary; for example, after the corneal epithelium location has been detected using the CCD 28, a location advanced forward by a prescribed distance towards the examined eye E could be designated as the forward movement location in the aforementioned Step Q3 or Step R3, or as the reversal location in the aforementioned Step S3. Specifically, light from the imaging light source 40 reflected by the examined eye E is received by the CCD 28 while advancing the apparatus optical system 10 forward towards the examined eye E. Then, the apparatus optical system 10 continues to advance until reflected light from the corneal epithelium is sensed by the CCD 28, whereupon the location at which the reflected light is sensed can be designated as the focal location of the corneal epithelium.

Also, while the preceding embodiments described a imaging operation in the direction of retraction of the apparatus optical system 10 away from the examined eye, it would of course be possible to carry out the imaging operation in the direction of forward advance of the apparatus optical system 10 closer to the examined eye after XY-alignment has been performed. Alternatively, by employing a sensor mechanism capable of highly accurate detection of the location of endothelial focus, to eliminate the need for multiple continuous imaging of the endothelium, and to obtain the objective endothelium image through endothelium imaging at a single point at the focal location.

In the preceding second and third embodiments, focus determinations could be carried out using a simple photoreceptor element like that shown in prior art Patent Citation 1, or using a CCD, in place of the line sensor 44.

During photographing of corneal endothelium images for XY-alignment purposes in Step Q5 or Step R4 in the preceding second and third embodiments, it will be possible to reduce the burden on the patient by using an infrared or other illuminating light source that does not emit visible light. During this process, imaging of the corneal endothelium for the purpose of observation will be carried out separately, and since corneal endothelium images for XY-alignment purposes photographed in Step Q5 or Step R4 have no effect on alignment in the Z-direction, as a general rule it is not necessary to consider correction by wavelength.

**Claims**

1. A cornea imaging apparatus (10) comprising:

   an illumination optical system (14) equipped with an illuminating light source (40) that emits a slit beam on a diagonal to an examined eye (E);
   a cornea imaging optical system (20) equipped with a photoelectric element (28) for receiving a reflected beam of light of the slit beam reflected from a cornea of the examined eye (E), and photographing an image of a corneal endothelium;
   Z-direction actuating means (116) for moving the illumination optical system (14) and the cornea imaging optical system (20) as a unit in a Z-direction which is a direction closer to and away from the examined eye (E);
   XY-direction actuating means (112, 114) for moving the illumination optical system (14) and the cornea imaging optical system (20) as a unit in a XY-directions orthogonal to the Z direction;
   a Z-direction location observation photoreceptor element (44) for sensing optical relative location in the Z-direction of the illumination optical system (14) and the cornea imaging optical system (20) relative to the examined eye (E) as the illumination optical system (14) and the cornea imaging optical system (20) move in the Z-direction;

XY-alignment pointer light emitting means (66) for emitting from a front side pointer light for a purpose of XY-alignment towards the examined eye (E);

an XY-alignment photoreceptor element (88) for receiving frontal reflection from a corneal epithelium of the examined eye (E) of the pointer light emitted by the XY-alignment pointer light emitting means (66);

**characterized in that** the apparatus further comprises:

a peripheral fixed gaze target (89) for selectively orienting a visual axis of the examined eye (E) in a plurality of directions on the diagonal with respect to straight ahead;

Z-direction location control means (117) for XY-alignment purposes that, with the visual axis of the examined eye (E) oriented in a diagonal direction by the peripheral fixed gaze target (89), on the basis of sensor results by the Z-direction location observation photoreceptor element (44) and using the Z-direction actuating means (116), adapted to move and position the illumination optical system (14) and the cornea imaging optical system (20) as a unit in a direction closer to the corneal endothelium beyond a focal point location with respect to the corneal epithelium of the examined eye (E);

XY-alignment optical information acquisition control means (17) that with the visual axis of the examined eye (E) oriented in the diagonal direction by the peripheral fixed gaze target (89), and positioned by the Z-direction location control means (117), using at least one of the cornea imaging optical system (20) and the Z-direction location observation photoreceptor element (44), adapted to acquire corneal endothelium optical information in the form of quantity of light distribution information or images of the corneal endothelium for the purpose of XY-alignment;

XY-alignment reference location determining means (Q7) that, using the corneal endothelium optical information acquired by the XY-alignment optical information acquisition control means (117) to calculate an orthogonal axis to a corneal endothelium of an examined eye, determines an XY-alignment reference location as a specific location in the XY-direction with consideration to an extent of deviation of an orthogonal axis to the corneal endothelium with respect to an orthogonal axis to the corneal epithelium sensed by the XY-alignment photoreceptor element (88); and

Corneal endothelium photographing image control means (117, S3-S5) that, on the basis of information of the XY-alignment reference location determined by the XY-alignment reference location determining means (Q7) and of optical information in the Z-direction sensed by the Z-direction location observation photoreceptor element (44), adapted to perform relative location control of the illumination optical system (14) and the cornea imaging optical system (20) with respect to the examined eye (E), and adapted to photograph the image of the corneal endothelium of the examined eye (E).

2. The cornea imaging apparatus (10) according to claim 1, wherein the XY-alignment optical information acquisition control means (117) acquires the corneal endothelium optical information on the optical axis of the frontal reflection from the corneal epithelium of the examined eye (E) of the XY-alignment pointer light directed thereon by the XY-alignment pointer light emitting means (66); and on the basis of the acquired corneal endothelium optical information, the XY-alignment reference location determining means (Q7) determines the XY-alignment reference location on a prescribed XY-direction line which corresponds to a diagonal direction of the visual axis.

3. The cornea imaging apparatus (10) according to claim 2, further comprising: XY-alignment reference location verification means that, after the XY-alignment reference location has been determined, moves and positions the illumination optical system (14) and the cornea imaging optical system (20) to the determined XY-alignment reference location, and with these positioned at the XY-alignment reference location, reacquires the corneal endothelium optical information using the XY-alignment optical information acquisition control means (117), and verifies the XY-alignment reference location on the basis of the corneal endothelium optical information acquired thereby.

4. The cornea imaging apparatus (10) according to claim 1, wherein the XY-alignment optical information acquisition control means (44) moves the illumination optical system (14) and the cornea imaging optical system (20) as a unit in a prescribed XY-direction which corresponds to the diagonal direction of the visual axis, and acquires the corneal endothelium optical information at multiple sites on a line in the prescribed XY-direction.

5. The cornea imaging apparatus (10) according to claim 4, wherein in the XY-alignment reference location determining means (Q7), acquisition of the corneal endothelium optical information by the XY-alignment optical information acquisition control means (117) on the line in the prescribed XY-direction is carried out until and terminated at a pre-established end location.

6. The cornea imaging apparatus (10) according to claim 4, in the XY-alignment reference location determining means (Q7), each time that the corneal endothelium optical information is acquired on the line in the prescribed XY-direction by the XY-alignment optical information acquisition control means (117), sequential evaluation is performed on the basis of the acquired corneal endothelium optical information; and when it is verified that an evaluation result meets a predetermined condition established in advance the XY-alignment reference location is determined and acquisition of the corneal endothelium optical information terminates.

7. The cornea imaging apparatus (10) according to any one of claims 4-6, wherein the Z-direction location observation photoreceptor element (44) comprises a line sensor, and in the XY-alignment optical information acquisition control means (117), the line sensor acquires corneal endothelium optical information for XY-alignment use from the line sensor, while in the XY-alignment optical information acquisition control means (117), a location of maximum quantity of reflected light in the line sensor is designated as the XY-alignment reference location.

8. The cornea imaging apparatus (10) according to claim 7, further comprising:

optical information correction level computing means that during error correction uses the line sensor in the XY-alignment optical information acquisition control means (117) to acquire corneal epithelium optical information in conjunction with acquisition of corneal endothelium optical information for XY-alignment purposes, and on the basis of the corneal epithelium optical information calculates a correction level for an error in the corneal endothelium optical information due to change in relative location in the Z-direction of the illumination optical system (14) and the cornea imaging optical system (20) with respect to the examined eye (E); and optical information correction executing means that corrects the XY-alignment corneal endothelium optical information acquired by the line sensor, using the correction level derived by the optical information correction level computing means.

9. The cornea imaging apparatus (10) according to claim 7 or 8, further comprising:

Z-direction change computing means that during acquisition of corneal endothelium optical information for XY-alignment purposes by the line sensor in the XY-alignment optical information acquisition control means (117) acquires corneal epithelium optical information with the line sensor, and on the basis of the corneal epithelium optical information calculates change of relative location in the Z-direction of the illumination optical system (14) and the cornea imaging optical system (20) with respect to the examined eye (E); and Z-direction location correction executing means that performs correction of relative location of the illumination optical system (14) and the cornea imaging optical system (20) with respect to the examined eye (E) in the Z-direction, using as the correction level the change of relative location in the Z-direction calculated by the Z-direction change computing means.

10. The cornea imaging apparatus (10) according to any one of claims 1-9, wherein the corneal endothelium photographing image control means (117, S3-S5) continuously photographs the corneal endothelium images at multiple sites along a line in the Z-direction while moving the illumination optical system (14) and the cornea imaging optical system (20) in the Z-direction relative to the examined eye (E).

11. The cornea imaging apparatus (10) according to claim 10, further comprising: XY-alignment correction control means that, during continuous photographing of corneal endothelium images at multiple sites along a line in the Z-direction, utilizes intervals between photographs in order to carry out photoreception of frontal reflection from the corneal epithelium with the XY-alignment pointer light emitting means (66) and the XY-alignment photoreceptor element (88) and calculate an orthogonal axis to the corneal epithelium, as well as to control the XY-direction actuating means (112, 114) while making allowance for the orthogonal axis to the corneal epithelium; and that performs position correction such that the illumination optical system (14) and the cornea imaging optical system (20) are positioned at the XY-alignment reference location determined by the XY-alignment reference location determining means (Q7).

12. A cornea imaging method that is executed by using the cornea imaging apparatus (10) according to any one of claims 1-11, comprising the steps of:

acquiring the corneal endothelium optical information in the form of quantity of light distribution information or images of the corneal endothelium for the purpose of XY-alignment with an XY-alignment optical information acquisition control means (117);
using the corneal endothelium optical information to calculate an orthogonal axis to a corneal endothelium of an examined eye (E), and designating the orthogonal axis as the XY-alignment reference location;
aligning the cornea imaging optical system (20) relative to the examined eye (E) in the XY-direction orthogonal to an orthogonal axis to the corneal endothelium so that the XY-alignment reference location is an imaging center of the cornea imaging optical system (20); and while relatively aligned in the XY-direction, photographing a peripheral portion of the corneal endothelium of the examined eye (E) with the cornea imaging optical system (20).

13. The cornea imaging method according to claim 12, further comprising the steps of:

continuously photographing multiple images of the corneal endothelium while moving the cornea imaging optical system (20) in the Z-direction closer to/away from the examined eye (E);
deriving the orthogonal axis to the corneal endothelium, in terms of the XY-alignment reference location indicating an amount of deviation in the XY-direction based on the orthogonal axis to a corneal epithelium of the examined eye (E); and
during intervals between photographing the multiple images of the corneal endothelium, sensing the orthogonal axis to the corneal epithelium, and performing relative alignment in the XY-direction so as to position the cornea imaging optical system (20) at the XY-alignment reference location with respect to the sensed orthogonal axis to the corneal epithelium.

**Patentansprüche**

1. Kornea-Abbildungsvorrichtung (10), umfassend:

ein optisches Belichtungssystem (14), welches mit einer Belichtungs-Lichtquelle (40) ausgestaltet ist, welche ein Schlitzstrahl auf einer Diagonalen zu einem untersuchten Auge (E) emittiert;
ein optisches Kornea-Abbildungssystem (20), welches mit einem fotoelektrischen Element (28) ausgestaltet ist, um einen reflektierten Lichtstrahl des Schlitzstrahls zu erfassen, welcher von einer Kornea des untersuchten Auges (E) reflektiert wird, und um ein Bild eines Kornea-Endothels zu fotografieren;
in Z-Richtung bewegende Mittel (116), um das optische Belichtungssystem (14) und das optische Kornea-Abbildungssystem (20) als eine Einheit in eine Z-Richtung zu bewegen, welche eine Richtung dichter zu und weg von dem untersuchten Auge (G) ist;
in XY-Richtung bewegende Mittel (112, 114), um das optische Belichtungssystem (14) und das optische Kornea-Abbildungssystem (20) als Einheit in eine XY-Richtung orthogonal zu der Z-Richtung zu bewegen;
ein in Z-Richtung lokalisierendes und beobachtendes Fotorezeptor-Element (44), um eine relative Position in der Z-Richtung des optischen Belichtungssystems (14) und des optischen Kornea-Abbildungssystems (20) relativ zu dem untersuchten Auge (E) optisch zu erfassen, wenn sich das optische Belichtungssystem (14) und das optische Kornea-Abbildungssystem (20) in der Z-Richtung bewegen;
Mittel (66) zum Emittieren eines Lichtpunkts zur XY-Ausrichtung, um von einer Vorderseite einen Lichtpunkt zum Zweck einer XY-Ausrichtung zu dem untersuchten Auge (E) zu emittieren;
ein XY-Ausrichtungs-Fotorezeptor-Element (88), um eine frontale Reflektion von einem Kornea-Epithel des untersuchten Auges (E) des Lichtpunkts, welcher durch die Mittel (66) zum Emittieren eines Lichtpunkts zur XY-Ausrichtung emittiert wird, zu erfassen;
**dadurch gekennzeichnet, dass** die Vorrichtung darüber hinaus umfasst:

ein im Umfang fixiertes Blickziel (89), um eine visuelle Achse des untersuchten Auges (E) in mehreren Richtungen auf der Diagonale bezüglich einer Geradeaus-Richtung selektiv auszurichten;
Steuermittel (117) zur Positionierung in Z-Richtung zum Zweck einer XY-Ausrichtung, welche, wobei die visuelle Achse des untersuchten Auges (E) durch das im Umfang fixierte Blickziel (89) in einer diagonalen Richtung ausgerichtet ist, abhängig von Sensorergebnissen durch das in Z-Richtung lokalisierende und beobachtende Fotorezeptor-Element (44) und mittels der in Z-Richtung bewegenden Mittel (116) ausgestaltet sind, um das optische Belichtungssystem (14) und das optische Kornea-Abbildungssystem (20) als eine Einheit in eine Richtung dichter an das Kornea-Endothel jenseits eines Brennpunkts bezüglich des Kornea-Epithels des untersuchten Auges (E) zu bewegen und zu positionieren;
Steuermittel (117) zur optischen Informationserfassung zur XY-Ausrichtung, welche, wobei die visuelle Achse des untersuchten Auges (E) durch das im Umfang fixierte Blickziel (89) in der diagonalen Richtung ausgerichtet ist und durch die Steuermittel (117) zur Lokalisierung in Z-Richtung positioniert ist, wobei das optische Kornea-Abbildungssystem und/oder das in Z-Richtung lokalisierende und beobachtende Fotorezeptor-Element (44) verwendet werden, ausgestaltet sind, um eine optische Information des Kornea-Endothels in der Form einer Quantität einer Lichtverteilungsinformation oder Bildern des Kornea-Endothels für den Zweck einer XY-Ausrichtung zu erfassen;

Referenzpositions-Bestimmungsmittel (Q7) zur XY-Ausrichtung, welche, unter Verwendung der optischen Information des Kornea-Endothels, die durch die Steuermittel (117) zur optischen Informationserfassung zur XY-Ausrichtung erfasst wird, um eine orthogonale Achse zu einem Kornea-Endothel eines untersuchten Auges zu berechnen, eine Referenzposition zur XY-Ausrichtung als eine bestimmte Position in der XY-Richtung unter Berücksichtigung eines Umfangs einer Abweichung einer orthogonalen Achse zu dem Kornea-Endothel bezüglich einer orthogonalen Achse zu dem Kornea-Epithel, welcher durch das XY-Ausrichtung-Fotorezeptor-Element (88) erfasst wird, bestimmen; und

Steuermittel (117, S3-S5) zum Fotografieren eines Bildes eines Kornea-Endothels, welche, abhängig von einer Information der Referenzposition zur XY-Ausrichtung, die durch die Referenzpositions-Bestimmungsmittel (Q7) zur XY-Ausrichtung bestimmt wird, und einer optischen Information in der Z-Richtung, die durch das in Z-Richtung lokalisierende und beobachtende Fotorezeptor-Element (44) erfasst wird, ausgestaltet sind, um eine Einstellung einer relativen Position des optischen Belichtungssystems (14) und des optischen Kornea-Abbildungssystems (20) bezüglich des untersuchten Auges (E) durchzuführen, und ausgestaltet sind, um das Bild des Kornea-Endothels des untersuchten Auges (E) zu fotografieren.

2. Kornea-Abbildungsvorrichtung (10) nach Anspruch 1, **dadurch gekennzeichnet, dass** die Steuermittel (117) zur optischen Informationserfassung zur XY-Ausrichtung die optische Information des Kornea-Endothels auf der optischen Achse der frontalen Reflektion von dem Kornea-Epithels des untersuchten Auges (E) des Lichtpunkts zur XY-Ausrichtung, welcher durch die Mittel (66) zum Emittieren eines Lichtpunkts zur XY-Ausrichtung darauf gerichtet ist, erfassen; und dass die Referenzpositions-Bestimmungsmittel (Q7) zur XY-Ausrichtung abhängig von der erfassten optischen Information des Kornea-Endothels die Referenzposition zur XY-Ausrichtung auf einer vorgeschriebenen Linie in XY-Richtung, welche mit einer diagonalen Richtung der visuellen Achse korrespondiert, bestimmen.

3. Kornea-Abbildungsvorrichtung (10) nach Anspruch 2, darüber hinaus umfassend: Referenzpositions-Verifikationsmittel zur XY-Ausrichtung, welche, nachdem die Referenzposition zur XY-Ausrichtung bestimmt worden ist, das optische Belichtungssystem (14) und das optische Kornea-Abbildungssystem (20) zu der bestimmten Referenzposition zur XY-Ausrichtung bewegen und dort positionieren, und wobei diese, welche an der Referenzposition zur XY-Ausrichtung positioniert sind, die optische Information des Kornea-Endothels mit Hilfe der Steuermittel (117) zur optischen Informationserfassung zur XY-Ausrichtung erneut erfassen und die Referenzposition zur XY-Ausrichtung abhängig von der optischen Information des Kornea-Endothels, welche dadurch erfasst wird, verifizieren.

4. Kornea-Abbildungsvorrichtung (10) nach Anspruch 1, **dadurch gekennzeichnet, dass** die Steuermittel (117) zur optischen Informationserfassung zur XY-Ausrichtung das optische Belichtungssystem (14) und das optische Kornea-Abbildungssystem (20) als eine Einheit in einer vorgeschriebenen XY-Richtung, welche mit der diagonalen Richtung der visuellen Achse korrespondiert, bewegen und die optische Information des Kornea-Endothels an mehreren Stellen auf einer Linie in der vorgeschriebenen XY-Richtung erfassen.

5. Kornea-Abbildungsvorrichtung (10) nach Anspruch 4, **dadurch gekennzeichnet, dass** in den Referenzpositions-Bestimmungsmitteln (Q7) zur XY-Ausrichtung ein Erfassen der optischen Information des Kornea-Endothels durch die Steuermittel (117) zur optischen Informationserfassung zur XY-Ausrichtung auf der Linie in der vorgeschriebenen XY-Richtung bis zu einer voreingestellten Endposition ausgeführt und dort beendet wird.

6. Kornea-Abbildungsvorrichtung (10) nach Anspruch 4, **dadurch gekennzeichnet, dass** in den Referenzpositions-Bestimmungsmitteln (Q7) zu XY-Ausrichtung zu jeder Zeit, zu welcher die optische Information des Kornea-Endothels auf der Linie in der vorgeschriebenen XY-Richtung durch die Steuermittel (117) zur optischen Informationserfassung zur XY-Ausrichtung erfasst werden, sequenziell eine Auswertung abhängig von der erfassten optischen Information des Kornea-Endothels ausgeführt wird; und dass, wenn verifiziert wird, dass ein Auswertungsergebnis eine vorbestimmte Bedingung, welche vorab festgelegt wurde, einhält, die Referenzposition zur XY-Ausrichtung bestimmt wird und dass Erfassen der optischen Information des Kornea-Endothels beendet wird.

7. Kornea-Abbildungsvorrichtung (10) nach einem der Ansprüche 4-6, **dadurch gekennzeichnet, dass** das in Z-Richtung lokalisierende und beobachtende Fotorezeptor-Element (44) einen Liniensensor umfasst, und dass in den Steuermitteln (117) zur optischen Informationserfassung zur XY-Ausrichtung der Liniensensor die optische Information des Kornea-Endothels zur XY-Ausrichtung erfasst während in den Steuermitteln (117) zur optischen Informationserfassung zur XY-Ausrichtung eine Position einer maximalen Quantität von reflektiertem Licht in dem Liniensensor als die Referenzposition zur XY-Ausrichtung bestimmt wird.

8. Kornea-Abbildungsvorrichtung (10) nach Anspruch 7, darüber hinaus umfassend:

Mittel zur Berechnung eines Korrekturwerts einer optischen Information, welche während einer Fehlerkorrektur den Liniensensor in den Steuermitteln (117) zur optischen Informationserfassung zur XY-Ausrichtung verwenden, um eine optische Information des Kornea-Epithels zusammen mit einer Erfassung einer optischen Information des Kornea-Endothels zum Zweck einer XY-Ausrichtung zu erfassen, und auf der Basis der optischen Information des Kornea-Epithels einen Korrekturwert für einen Fehler in der optischen Information des Kornea-Endothels während einer Änderung bezüglich der relativen Position des optischen Belichtungssystems (14) und des optischen Kornea-Abbildungssystems (20) in der Z-Richtung bezüglich des untersuchten Auges (E) berechnen; und
Mittel zur Ausführung einer Korrektur einer optischen Information, welche die optische Information des Kornea-Endothels zur XY-Ausrichtung, die durch den Liniensensor erfasst wird, mit Hilfe des Korrekturwerts, welcher durch die Mittel zur Berechnung eines Korrekturwerts einer optischen Information bestimmt wird, korrigieren.

9. Kornea-Abbildungsvorrichtung (10) nach Anspruch 7 oder 8, darüber hinaus umfassend:

Mittel zur Berechnung einer Änderung in Z-Richtung, welche während einem Erfassen einer optischen Information des Kornea-Endothels für eine XY-Ausrichtung durch den Liniensensor in den Steuermitteln (117) zur optischen Informationserfassung zur XY-Ausrichtung eine optische Information des Kornea-Epithels mit dem Liniensensor erfassen und abhängig von der optischen Information des Kornea-Epithels eine Änderung der relativen Position in der Z-Richtung des optischen Belichtungssystems (14) und des optischen Kornea-Abbildungssystems (20) bezüglich des untersuchten Auges (E) berechnen; und
Mittel zur Ausführung einer Positionskorrektur in Z-Richtung, welche eine Korrektur der relativen Position des optischen Belichtungssystems (14) und des optischen Kornea-Abbildungssystems (20) bezüglich des untersuchten Auges (E) in der Z-Richtung durchführen, wobei als der Korrekturwert die Änderung der relativen Position in der Z-Richtung, welche durch die Mittel zur Berechnung der Änderung in Z-Richtung berechnet wird, verwendet wird.

10. Kornea-Abbildungsvorrichtung (10) nach einem der Ansprüche 1-9, **dadurch gekennzeichnet, dass** die Steuermittel (117, S3-S5) zum Fotografieren eines Bildes eines Kornea-Endothels Bilder des Kornea-Endothels an mehreren Stellen entlang einer Linie in der Z-Richtung ununterbrochen fotografieren während das optische Belichtungssystem (14) und das optische Kornea-Abbildungssystem (20) in der Z-Richtung relativ zu dem untersuchten Auge (E) bewegt wird.

11. Kornea-Abbildungsvorrichtung (10) nach Anspruch 10, darüber hinaus umfassend: Korrektursteuermittel zur XY-Ausrichtung, welche, während Bilder des Kornea-Endothels an mehreren Stellen entlang einer Linie in der Z-Richtung ununterbrochen fotografiert werden, Intervalle zwischen Fotografien verwenden, um eine Fotoaufnahme einer frontalen Reflektion von dem Kornea-Epithel mit Hilfe der Mittel (66) zum Emittieren eines Lichtpunkts zur XY-Ausrichtung und des XY-Ausrichtungs-Fotorezeptor-Elements (88) auszuführen und eine orthogonal Achse zu dem Kornea-Epithel zu berechnen, um auch die in XY-Richtung bewegenden Mittel (112, 114) zu steuern, während eine Toleranz für die orthogonale Achse zu dem Kornea-Epithel berücksichtigt wird; und welche eine Positionskorrektur durchführen, so dass das optische Belichtungssystem (14) und das optische Kornea-Abbildungssystem (20) an der Referenzposition zur XY-Ausrichtung positioniert sind, welche durch die Referenzpositions-Bestimmungsmittel (Q7) zur XY-Ausrichtung bestimmt wird.

12. Kornea-Abbildungsverfahren, welches unter Verwendung der Kornea-Abbildungsvorrichtung (10) nach einem der Ansprüche 1-11 ausgeführt wird und welches die Schritte umfasst:

Erfassen einer optischen Information des Kornea-Endothels in der Form einer Quantität einer Lichtverteilungsinformation oder Bildern des Kornea-Endothels zum Zweck einer XY-Ausrichtung mit den Steuermitteln (117) zur optischen Informationserfassung zur XY-Ausrichtung;
Verwenden der optischen Information des Kornea-Endothels, um eine orthogonale Achse zu einem Kornea-Endothel eines untersuchten Auges (E) zu berechnen und die orthogonale Achse als die Referenzposition zur XY-Ausrichtung zu bestimmen;
Ausrichten des optischen Kornea-Abbildungssystems (20) relativ zu dem untersuchten Auge (E) in der XY-Richtung orthogonal zu einer orthogonalen Achse zu dem Kornea-Endothel, so dass die Referenzposition zur XY-Ausrichtung ein Abbildungszentrum des optischen Kornea-Abbildungssystems (20) ist; und
während einer relativen Ausrichtung in der XY-Richtung Fotografieren eines Umfangsabschnitts des Kornea-Endothels des untersuchten Auges (E) mit dem optischen Kornea-Abbildungssystem (20).

**13.** Kornea-Abbildungsverfahren nach Anspruch 12, darüber hinaus die Schritte umfassend:

Ununterbrochenes Fotografieren von mehreren Bildern des Kornea-Endothels während das optische Kornea-Abbildungssystem (20) in der Z-Richtung dichter zu dem untersuchten Auge (E) oder weiter von diesem weg bewegt wird;
Ableiten der orthogonalen Achse zu dem Kornea-Endothel bezüglich der Referenzposition zur XY-Ausrichtung, welche einen Abweichungsumfang in der XY-Richtung abhängig von der orthogonalen Achse zu einem Kornea-Epithel des untersuchten Auges (E) anzeigt; und
während Intervallen zwischen einem Fotografieren der mehreren Bilder des Kornea-Endothels Erfassen der orthogonalen Achse zu dem Kornea-Epithel und Ausführen einer relativen Ausrichtung in der XY-Richtung, um so das optische Kornea-Abbildungssystem (20) an der Referenzposition zur XY-Ausrichtung bezüglich der erfassten orthogonalen Achse zu dem Kornea-Epithel zu positionieren.

**Revendications**

**1.** Appareil d'imagerie de la cornée (10) comprenant :

un système optique d'éclairage (14) équipé d'une source de lumière d'éclairage (40) qui émet un faisceau en fente en diagonale vers un oeil examiné (E) ;
un système optique d'imagerie de la cornée (20) équipé d'un élément photoélectrique (28) pour recevoir un faisceau lumineux réfléchi du faisceau en fente réfléchi par une cornée de l'oeil examiné (E), et prendre une image photographique d'un endothélium cornéen ;
un moyen d'actionnement de direction Z (116) pour déplacer le système optique d'éclairage (14) et le système optique d'imagerie de la cornée (20) en un bloc dans une direction Z qui est une direction qui se rapproche et s'éloigne de l'oeil examiné (E) ;
un moyen d'actionnement de direction XY (112, 114) pour déplacer le système optique d'éclairage (14) et le système optique d'imagerie de la cornée (20) en un bloc dans une direction XY orthogonale à la direction Z ;
un élément photorécepteur d'observation de position de direction Z (44) pour détecter la position optique relative dans la direction Z du système optique d'éclairage (14) et du système optique d'imagerie de la cornée (20) par rapport à l'oeil examiné (E) pendant que le système optique d'éclairage (14) et le système optique d'imagerie de la cornée (20) se déplacent dans la direction Z ;
un moyen émettant une lumière de pointage d'alignement XY (66) pour émettre depuis un pointeur latéral avant une lumière destinée à l'alignement XY vers l'oeil examiné (E) ;
un élément photorécepteur d'alignement XY (88) pour recevoir une réflexion frontale d'un endothélium cornéen de l'oeil examiné (E) de la lumière de pointage émise par le moyen émettant une lumière de pointage d'alignement XY (66) ;
**caractérisé en ce que** l'appareil comprend en outre :

une cible de visée fixe périphérique (89) pour orienter sélectivement un axe visuel de l'oeil examiné (E) dans une pluralité de directions sur la diagonale par rapport à la direction droit devant ;
un moyen de commande de position de direction Z (117) permettant un alignement XY qui, avec l'axe visuel de l'oeil examiné (E) orienté selon une direction diagonale par la cible de visée fixe périphérique (89), d'après des résultats de capteur fournis par l'élément photorécepteur d'observation de position de direction Z (44) et en utilisant le moyen d'actionnement de direction Z (116), est adapté pour déplacer et positionner le système optique d'éclairage (14) et le système optique d'imagerie de la cornée (20) en un bloc dans une direction qui se rapproche de l'endothélium cornéen au-delà d'une position de point focal par rapport à l'épithélium cornéen de l'oeil examiné (E) ;
un moyen de commande d'acquisition d'information optique d'alignement XY (117) qui, avec l'axe visuel de l'oeil examiné (E) orienté selon la direction diagonale par la cible de visée fixe périphérique (89), et positionné par le moyen de commande de position de direction Z (117), en utilisant au moins un élément parmi le système optique d'imagerie de la cornée (20) et l'élément photorécepteur d'observation de position de direction Z (44), est adapté pour acquérir une information optique d'endothélium cornéen sous la forme d'une information de distribution de quantité de lumière ou d'images de l'endothélium cornéen pour permettre l'alignement XY ;

un moyen de détermination de position de référence d'alignement XY (Q7) qui, en utilisant l'information optique d'endothélium cornéen acquise par le moyen de commande d'acquisition d'information optique d'alignement XY (117) pour calculer un axe orthogonal à un endothélium cornéen d'un oeil examiné, détermine une position de référence d'alignement XY comme position spécifique dans la direction XY relativement à un degré d'écart d'un axe orthogonal à l'endothélium cornéen par rapport à un axe orthogonal à l'épithélium cornéen mesuré par l'élément photorécepteur d'alignement XY (88) ; et

un moyen de commande d'image photographique d'endothélium cornéen (117, S3-S5) qui, d'après l'information de la position de référence d'alignement XY déterminée par le moyen de détermination de position de référence d'alignement XY (Q7) et d'après une information optique dans la direction Z mesurée par l'élément photorécepteur d'observation de position de direction Z (44), est adapté pour réaliser une commande de position relative du système optique d'éclairage (14) et du système optique d'imagerie de la cornée (20) par rapport à l'oeil examiné (E), et est adapté pour prendre une image photographique de l'endothélium cornéen de l'oeil examiné (E).

2. Appareil d'imagerie de la cornée (10) selon la revendication 1, dans lequel le moyen de commande d'acquisition d'information optique d'alignement XY (117) acquiert l'information optique d'endothélium cornéen sur l'axe optique de la réflexion frontale produite par l'épithélium cornéen de l'oeil examiné (E) de la lumière de pointage d'alignement XY dirigée sur celui-ci par le moyen émettant une lumière de pointage d'alignement XY (66) ; et en se basant sur l'information optique d'endothélium cornéen acquise, le moyen de détermination de position de référence d'alignement XY (Q7) détermine la position de référence d'alignement XY sur une ligne de direction XY imposée qui correspond à une direction diagonale de l'axe visuel.

3. Appareil d'imagerie de la cornée (10) selon la revendication 2, comprenant en outre : un moyen de vérification de position de référence d'alignement XY qui, après que la position de référence d'alignement XY a été déterminée, déplace et positionne le système optique d'éclairage (14) et le système optique d'imagerie de la cornée (20) jusqu'à la position de référence d'alignement XY déterminée, et avec ceux-ci positionnés à la position de référence d'alignement XY, acquiert de nouveau l'information optique d'endothélium cornéen en utilisant le moyen de commande d'acquisition d'information optique d'alignement XY (117), et vérifie la position de référence d'alignement XY d'après l'information optique d'endothélium cornéen ainsi acquise.

4. Appareil d'imagerie de la cornée (10) selon la revendication 1, dans lequel le moyen de commande d'acquisition d'information optique d'alignement XY (44) déplace le système optique d'éclairage (14) et le système optique d'imagerie de la cornée (20) en un bloc dans une direction XY imposée qui correspond à la direction diagonale de l'axe visuel, et acquiert l'information optique d'endothélium cornéen en plusieurs sites sur une ligne dans la direction XY imposée.

5. Appareil d'imagerie de la cornée (10) selon la revendication 4, dans lequel, dans le moyen de détermination de position de référence d'alignement XY (Q7), l'acquisition de l'information optique d'endothélium cornéen par le moyen de commande d'acquisition d'information optique d'alignement XY (117) sur la ligne dans la direction XY imposée est exécutée jusqu'à une position de fin préétablie où elle est arrêtée.

6. Appareil d'imagerie de la cornée (10) selon la revendication 4, dans lequel, dans le moyen de détermination de position de référence d'alignement XY (Q7), à chaque fois que l'information optique d'endothélium cornéen est acquise sur la ligne dans la direction XY imposée par le moyen de commande d'acquisition d'information optique d'alignement XY (117), une évaluation séquentielle est réalisée d'après l'information optique d'endothélium cornéen acquise ; et quand il est vérifié qu'un résultat d'évaluation remplit une condition prédéterminée établie à l'avance, la position de référence d'alignement XY est déterminée et l'acquisition de l'information optique d'endothélium cornéen est arrêtée.

7. Appareil d'imagerie de la cornée (10) selon l'une quelconque des revendications 4 à 6, dans lequel l'élément photorécepteur d'observation de position de direction Z (44) comprend un capteur de ligne, et dans le moyen de commande d'acquisition d'information optique d'alignement XY (117), le capteur de ligne acquiert une information optique d'endothélium cornéen pour permettre l'alignement XY à partir du capteur de ligne, tandis que dans le moyen de commande d'acquisition d'information optique d'alignement XY (117), une position de quantité maximale de lumière réfléchie dans le capteur de ligne est désignée comme étant la position de référence d'alignement XY.

8. Appareil d'imagerie de la cornée (10) selon la revendication 7, comprenant en outre :

un moyen de calcul de niveau de correction d'information optique qui, pendant une correction d'erreur, utilise le capteur de ligne dans le moyen de commande d'acquisition d'information optique d'alignement XY (117) pour acquérir une information optique d'épithélium cornéen en association avec l'acquisition d'une information optique d'endothélium cornéen pour permettre un alignement XY, et qui, d'après l'information optique d'épithélium cornéen, calcule un niveau de correction pour une erreur dans l'information optique d'endothélium cornéen due à un changement de la position relative dans la direction Z du système optique d'éclairage (14) et du système optique d'imagerie de la cornée (20) par rapport à l'oeil examiné (E) ; et un moyen d'exécution de correction d'information optique qui corrige l'information optique d'endothélium cornéen d'alignement XY acquise par le capteur de ligne, en utilisant le niveau de correction obtenu par le moyen de calcul de niveau de correction d'information optique.

**9.** Appareil d'imagerie de la cornée (10) selon la revendication 7 ou 8, comprenant en outre :

un moyen de calcul de changement de direction Z qui, pendant l'acquisition d'une information optique d'endothélium cornéen destinée à l'alignement XY par le capteur de ligne dans le moyen de commande d'acquisition d'information optique d'alignement XY (117), acquiert une information optique d'épithélium cornéen avec le capteur de ligne, et qui d'après l'information optique d'épithélium cornéen, calcule un changement de position relative dans la direction Z du système optique d'éclairage (14) et du système optique d'imagerie de la cornée (20) par rapport à l'oeil examiné (E) ; et
un moyen d'exécution de correction de position de direction Z qui effectue une correction de la position relative du système optique d'éclairage (14) et du système optique d'imagerie de la cornée (20) par rapport à l'oeil examiné (E) dans la direction Z, en utilisant comme niveau de correction le changement de position relative dans la direction Z calculé par le moyen de calcul de changement de direction Z.

**10.** Appareil d'imagerie de la cornée (10) selon l'une quelconque des revendications 1 à 9, dans lequel le moyen de commande d'image photographique d'endothélium cornéen (117, S3-S5) prend de façon continue des images photographiques de l'endothélium cornéen en plusieurs sites le long d'une ligne dans la direction Z tout en déplaçant le système optique d'éclairage (14) et le système optique d'imagerie de la cornée (20) dans la direction Z par rapport à l'oeil examiné (E).

**11.** Appareil d'imagerie de la cornée (10) selon la revendication 10, comprenant en outre : un moyen de commande de correction d'alignement XY qui, pendant la prise continue d'images photographiques de l'endothélium cornéen en plusieurs sites le long d'une ligne dans la direction Z, utilise des intervalles entre photographies afin de réaliser une photoréception d'une réflexion frontale provenant de l'épithélium cornéen avec le moyen émettant une lumière de pointage d'alignement XY (66) et l'élément photorécepteur d'alignement XY (88) et de calculer un axe orthogonal à l'épithélium cornéen, ainsi que pour commander le moyen d'actionnement de direction XY (112, 114) tout en prévoyant une marge pour l'axe orthogonal à l'épithélium cornéen ; et qui réalise une correction de position de telle manière que le système optique d'éclairage (14) et le système optique d'imagerie de la cornée (20) sont positionnés à la position de référence d'alignement XY déterminée par le moyen de détermination de position de référence d'alignement XY (Q7).

**12.** Procédé d'imagerie de la cornée qui est exécuté en utilisant l'appareil d'imagerie de la cornée (10) selon l'une quelconque des revendications 1 à 11, comprenant les étapes suivantes :

acquérir l'information optique d'endothélium cornéen sous la forme d'une information de distribution de quantité de lumière ou d'images de l'endothélium cornéen pour permettre l'alignement XY avec un moyen de commande d'acquisition d'information optique d'alignement XY (117) ;
utiliser l'information optique d'endothélium cornéen pour calculer un axe orthogonal à un endothélium cornéen d'un oeil examiné (E), et désigner l'axe orthogonal comme position de référence d'alignement XY ;
aligner le système optique d'imagerie de la cornée (20) par rapport à l'oeil examiné (E) dans la direction XY orthogonale à un axe orthogonal à l'endothélium cornéen de telle manière que la position de référence d'alignement XY est un centre d'imagerie du système optique d'imagerie de la cornée (20) ; et tout en étant aligné relativement dans la direction XY, photographier une partie périphérique de l'endothélium cornéen de l'oeil examiné (E) avec le système optique d'imagerie de la cornée (20).

**13.** Procédé d'imagerie de la cornée selon la revendication 12, comprenant en outre les étapes suivantes :

prendre de façon continue plusieurs images photographiques de l'endothélium cornéen tout en rapprochant et éloignant le système optique d'imagerie de la cornée (20) de l'oeil examiné (E) dans la direction Z ;
obtenir l'axe orthogonal à l'endothélium cornéen, relativement à la position de référence d'alignement XY indiquant un degré d'écart dans la direction XY basé sur l'axe orthogonal à un épithélium cornéen de l'oeil examiné (E) ; et
pendant les intervalles entre la prise d'images photographiques de l'endothélium cornéen, détecter l'axe orthogonal à l'épithélium cornéen, et effectuer un alignement relatif dans la direction XY afin de positionner le système optique d'imagerie de la cornée (20) à la position de référence d'alignement XY par rapport à l'axe orthogonal à l'épithélium cornéen détecté.

# FIG.1

# FIG.2

89a

89f

$0_1$

89b

89c

89e

89d

# FIG.3

100

Y

Z

X

106

110

104

102

108

# FIG.4

# FIG.5

```
        ┌─────────────┐
        │    START    │
        └──────┬──────┘
               │
        ┌──────▼──────────┐
   S1   │  XY ALIGNMENT   │
        │ SENSOR CIRCUIT  │
        └──────┬──────────┘
               │
        ┌──────▼──────┐
   S2   │   FORWARD   │
        │  OPERATION  │
        └──────┬──────┘
               │
        ┌──────▼──────────────┐
   S3   │ REVERSAL·RETRACTION │
        │ OPERATION INITIATED │
        └──────┬──────────────┘
               │
        ┌──────▼──────────┐
   S4   │ LIGHT EMISSION  │
        └──────┬──────────┘
               │
        ┌──────▼──────────────────────────────┐
   S5   │ ENDOTHELIAL POSITION SENSING         │
        │ ·IMAGING· SELECTION OF IMAGES        │
        └──────┬───────────────────────────────┘
               │
        ┌──────▼──────┐
   S6   │   IMAGING   │
        │ TERMINATED  │
        └──────┬──────┘
               │
        ┌──────▼──────┐
        │     END     │
        └─────────────┘
```

# FIG.6

E     126     110

125

# FIG.7

```
            ┌──────────────────────┐
            │        Start         │
            └──────────────────────┘
                        │
 Q1                     ▼
 ┌──────────────────────────────────┐
 │   FIXED  GAZE  LAMPS  SELECTED    │
 └──────────────────────────────────┘
                        │
 Q2                     ▼
 ┌──────────────────────────────────┐
 │ REFERENCE  LOCATION  (PROVISIONAL) │
 │         XY  ALIGNMENT             │
 └──────────────────────────────────┘
                        │
 Q3                     ▼
 ┌──────────────────────────────────────┐
 │ FORWARD  ADVANCE  TOWARD  ENDOTHELIAL SIDE │
 │ ACROSS  CORNEAL  EPITHELIUM  FOCAL LOCATION │
 └──────────────────────────────────────┘
                        │
 Q4                     ▼
 ┌──────────────────────────────────┐
 │     PRESCRIBED  DISTANCE          │
 │   MOVEMENT  WITHIN  XY  PLANE     │
 └──────────────────────────────────┘
                        │
 Q5                     ▼
 ┌──────────────────────────────────┐
 │  LINE  SENSOR  REFLECTED  LIGHT   │
 │    FROM  EPITHELIUM  SENSED       │
 └──────────────────────────────────┘
                        │
 Q6                     ▼
          ◇ TERMINAL  SET ◇  — N —┐
          ◇ POSITION  REACHED? ◇  │
                    │ Y
                    ▼
 Q7
 ┌──────────────────────────────────┐
 │  REFERENCE  LOCATION  FOR         │
 │  XY  ALIGNMENT  DETERMINED        │
 └──────────────────────────────────┘
                        │
 Q8                     ▼
 ┌──────────────────────────────────────────────┐
 │ XYZ  OPERATAED  SIMULTANEOUSLY  TO  PROTOMERITE │
 │      XY:  XY  ALIGNMENT  REFERENCE  LOCATION   │
 │ Z:  PRESCRIBED  DISTANCE  (FIXED VALUE)  FORWARDED │
 └──────────────────────────────────────────────┘
                        │
 Q9                     ▼
 ┌──────────────────────────────────┐
 │   XY  ALIGNMENT  TERMINATION      │
 │       SIGNAL  OUTPUT              │
 └──────────────────────────────────┘
                        │
                        ▼
            ┌──────────────────────┐
            │         End          │
            └──────────────────────┘
```

# FIG.8

Y DIRECTION

DIRECTION OF GAZE

P end

P 3

P 2

X DIRECTION

P 1

P 0

# FIG.9

LIGHT QUANTITY

128

130

130

128

$\underrightarrow{500\,\mu\,m}$

$P_3$
$P_{end}$
$P_2$
$P_1$
$P_0$

EPITHELIUM
FOCAL
LOCATION

Z DIRECTION LOCATION
ADJUSTMENT REFERENCE
(EPITHELIUM REFLECTION)

LINE SENSOR LOCATION

# FIG.10

LUMINANCE VALUE

250

200

150

100

50

0

$\Delta C_0$
$(\Delta C_i)$

$\Delta C_0$
$(\Delta C_i)$

$C_0$

$C_0$

-500    -300    -100    100    300    500

AMOUNT OF Z DIRECTION SHIFT FROM REFERENCE (FOCAL) POINT $\Delta Z(\mu\,m)$

# FIG.11

70μm SHIFT UPWARD
FROM INITIAL LOCATION

210μm SHIFT UPWARD
FROM INITIAL LOCATION

350μm SHIFT UPWARD
FROM INITIAL LOCATION

# FIG.12

XY SHIFT LOCATION

# FIG.13

```
                    ┌──────────────────┐
                    │      Start       │
                    └──────────────────┘
                              │
  R1                          ▼
        ┌──────────────────────────────┐
        │       FIXED GAZE LAMPS        │
        │          SELECTED             │
        └──────────────────────────────┘
                              │
  R2                          ▼
        ┌──────────────────────────────┐
        │      REFERENCE  LOCATION      │
        │  (PROVISIONAL)  XY  ALIGNMENT │
        └──────────────────────────────┘
                              │
  R3                          ▼
   ┌─────────────────────────────────────────────┐
   │  FORWARD ADVANCE TOWARD ENDOTHELIAL SIDE      │
   │  ACROSS CORNEAL EPITHELIUM FOCAL LOCATION     │
   └─────────────────────────────────────────────┘
                              │
  R4                          ▼
   ┌─────────────────────────────────────────────┐
   │  PHOTOGRAPHING CORNEAL ENDOTHELIAL IMAGE      │
   │            FOR XY ALIGNMENT                   │
   └─────────────────────────────────────────────┘
                              │
  R5                          ▼
        ┌──────────────────────────────┐
        │  CALCULATING REFERENCE LOCATION│
        │        FOR XY ALIGNMENT        │
        └──────────────────────────────┘
                              │
  R6                          ▼
 ┌───────────────────────────────────────────────────┐
 │  XYZ  OPERATAED  SIMULTANEOUSLY  TO  PROTOMERITE    │
 │        XY:  XY  ALIGNMENT  REFERENCE  LOCATION       │
 │  Z:  PRESCRIBED  DISTANCE  (FIXED VALUE)  FORWARDED  │
 └───────────────────────────────────────────────────┘
                              │
  R7                          ▼
        ┌──────────────────────────────┐
        │  XY  ALIGNMENT  TERMINATION   │
        │        SIGNAL  OUTPUT         │
        └──────────────────────────────┘
                              │
                              ▼
                    ┌──────────────────┐
                    │       End        │
                    └──────────────────┘
```

## FIG.14A

EXAMINED EYE IMAGE

CORNEAL IMAGE

MONITOR SCREEN

## FIG.14B

$\alpha$

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2812421 B
- JP 3338529 B
- JP 2007028039 A
- EP 0628281 A
- US 5757461 A
- JP 6063018 A
- JP 8071043 A
- JP 7121255 B